# EUROPÄISCHE PATENTSCHRIFT

(11) **EP 3 649 099 B1**
(45) Veröffentlichungstag und Bekanntmachung des Hinweises auf die Patenterteilung: **07.06.2023**
(21) Anmeldenummer: 18730019.9
(22) Anmeldetag: 19.06.2018
(51) Int. Cl.: C07C 67/26, C07C 69/54, C08F 222/10, C04B 40/06

(54) **EPOXYMETHACRYLAT-VERBINDUNGEN UND DEREN VERWENDUNG**
EPOXY METHACRYLATE COMPOUNDS AND THEIR USE
COMPOSÉS DE MÉTHACRYLATE D'ÉPOXY ET LEUR UTILISATION

(30) Priorität: 03.07.2017 EP 17179290
(43) Veröffentlichungstag der Anmeldung: 13.05.2020
(73) Patentinhaber: Hilti Aktiengesellschaft, 9494 Schaan (LI)
(72) Erfinder: NICKERL, Georg, 86911 Dießen am Ammersee (DE); GNASS, Beate, 86368 Gersthofen (DE); BUNZEN, Jens, 86159 Augsburg (DE); GAEFKE, Gerald, 86159 Augsburg (DE); BÜRGEL, Thomas, 86899 Landsberg (DE)
(74) Vertreter: Hilti Aktiengesellschaft Corporate Intellectual Property
(86) Internationale Anmeldenummer: PCT/EP2018/066215
(87) Internationale Veröffentlichungsnummer: WO 2019/007666

(56) Entgegenhaltungen:
- EP-A1- 0 713 015
- EP-A1- 2 922 890
- WO-A1-2017/174438
- WO-A1-2017/174443
- WO-A1-2017/174444
- GB-A- 1 006 587

## Beschreibung

Die Erfindung betrifft die Verwendung von niedrigviskosen Epoxymethacrylat-Verbindungen als Backbone-Harze in Reaktivharzen, insbesondere zur Erniedrigung der Viskosität solcher Verbindungen enthaltenden Reaktivharzen und damit der Auspresskräfte von daraus hergestellten Reaktivharzkomponenten. Des Weiteren betrifft die Erfindung die Verwendung dieser Reaktivharze und der deren Reaktivharzkomponenten zur chemischen Befestigung von Verankerungsmitteln in Aussparungen.

Die derzeit eingesetzten radikalisch härtbaren Befestigungsmassen basieren auf ungesättigten Polyestern, Vinylesterurethanharzen und Epoxyacrylaten. Es handelt sich dabei meist um Zweikomponenten-Reaktivharz-Systeme, wobei eine Komponente das Harz (sogenannte Komponente (A)) und die andere Komponente (Komponente (B)) das Härtungsmittel enthält. Weitere Bestandteile wie anorganische Füllmittel und Additive, Beschleuniger, Stabilisatoren und Reaktivverdünner können in der einen und/oder der anderen Komponente enthalten sein. Durch Vermischen der beiden Komponenten wird die Härtung der gemischten Komponenten in Gang setzt. Bei Verwendung der Befestigungsmassen zur Befestigung von Verankerungselementen in Bohrlöchern erfolgt die Härtung in den Bohrlöchern.

Eine solche Befestigungsmasse ist beispielsweise aus der DE 3940138 A1 bekannt. Diese beschreibt Befestigungsmassen auf Basis von cycloaliphatische Gruppen tragenden Monomeren, die zusätzlich ungesättigte Polyester- oder Vinylesterharze enthalten können. Derartige Mörtelmassen weisen aber verhältnismäßig hohe Viskositäten auf, wodurch ihre Anwendung, vor allem für die chemische Befestigungstechnik beschränkt wird.

Aus der EP 0713015 A1 ist eine selbsttragende und lagerstabile Dübelmasse für die chemische Befestigungstechnik bekannt, die direkt in das Bohrloch eingesetzt werden kann. Die Dübelmasse ist derart verpackt, dass der Radikalinitiator oder der Beschleuniger als separate Phase, räumlich getrennt von dem radikalisch polymerisierbaren Reaktionsharz eingelagert ist.

Auf Baustellen können relativ große Temperaturbereiche von beispielsweise -25°C bis +45°C auftreten, je nach Jahreszeit und/oder geographischer Lage. Daher kann die hohe Viskosität bei den eingangs beschriebenen härtbaren Befestigungsmassen und deren resultierendes thixotropes Verhalten bei der Anwendung zu Problemen führen. Daher werden große Anforderungen an den Einsatzbereich, insbesondere den Einsatz in verschiedenen Temperaturbereichen, derartiger Befestigungsmassen gestellt.

Zum einen sollte im niedrigen Temperaturbereich eine ausreichend niedrige Viskosität der Masse beim Auspressen gewährleistet werden, so dass die Masse einen nicht zu hohen Strömungswiderstand aufweist. Damit soll sichergestellt werden, dass die Massen beispielsweise mit einem Handdispenser verarbeitet, z.B. in das Bohrloch injiziert werden kann. Insbesondere bei der Verwendung von Statikmischern ist eine niedrige Viskosität für eine einwandfreie Vermischung der beiden Komponenten von Bedeutung.

Zum anderen sollte die Masse im höheren Temperaturbereich hinreichend standfest sein, so dass ein Nachlaufen der einzelnen Komponenten nach dem Entspannen des Dispensers verhindert wird sowie bei der Überkopf-Montage die Masse nicht aus dem Bohrloch herausläuft.

Ein weiteres durch Temperaturschwankungen bedingtes Problem ist, dass die radikalische Kettenpolymerisation nicht einheitlich abläuft. Die ausgehärtete Befestigungsmasse weist somit eine schwankende/unregelmäßige und häufig unzureichende Homogenität aus, was sich in Schwankungen der Lastwerte und häufig auch in generell niedrigen Lastwerten wiederspiegelt. Beispielsweise kann es bei Temperaturen unter 20°C durch eine Erhöhung der Viskosität zu einem frühzeitigen Erstarren der Befestigungsmasse kommen. Dadurch fällt der Umsatz bei der radikalischen Kettenpolymerisation wesentlich geringer aus, was zu einer Verringerung der Lastwerte beiträgt.

Da Temperaturschwankungen auf der Baustelle sich nicht vermeiden lassen, besteht nach wie vor ein Bedarf an Zweikomponenten-Reaktivharz-Systemen, welche sowohl bei hohen als auch bei niedrigen Temperaturen die Homogenität und die damit verbundene Reproduzierbarkeit der Lastwerte gewährleisten.

Um oben genannte Probleme zu adressieren, wird bei den auf dem Markt befindlichen Befestigungsmassen der Anteil an Reaktivverdünnern erhöht, was letztendlich zu einer Verringerung des Harzanteils in der Masse führt. Nicht selten beträgt der Anteil an Reaktivverdünnern mindestens 50%, bezogen auf das Reaktivharz.

Die Erhöhung des Anteils an Reaktivverdünnern führt allerdings auch zu einigen Nachteilen, die sich vor allem bei der Anwendung der Befestigungsmasse zur Befestigung von Verankerungsmittel in Bohrlöchern bemerkbar machen.

Ein erheblicher Nachteil ist, dass die Verringerung des Anteils an hochviskosem Harz, welches für die Leistungsfähigkeit der Masse wesentlich ist, die Leistungsfähigkeit der ausgehärteten Befestigungsmasse negativ beeinflusst.

Ein weiterer Nachteil ist ein größerer Schrumpf der Befestigungsmasse nach dem Aushärten, was zusätzlich die Leistungsfähigkeit der ausgehärteten Befestigungsmasse negativ beeinflussen kann. Dies wird darauf zurückgeführt, dass der Kontakt zwischen der ausgehärteten Befestigungsmasse und den beim Erstellen des Bohrlochs in der Bohrlochwandung gebildeten Hinterschnitten, die insbesondere bei der Verwendung von Schlagbohrmaschinen auftreten, deutlich verringert wird. Dies verhindert meist auch die Anwendung der Befestigungsmassen auf Basis radikalisch härtbarer Verbindungen in diamantgebohrten Löchern.

Ein weiterer Nachteil ist, dass sich je nach Art des Reaktivverdünners der Anteil an flüchtigen organischen Verbindungen (VOC; Volatile Organic Compounds) in den Massen erhöhen kann. Dies kann zu Ausdünstungen aus der Befestigungsmasse und/oder der Kartusche führen und gegebenenfalls zu einem daraus resultierenden Leistungsabfall der ausgehärteten Befestigungsmasse. Einige dieser Verbindungen können zudem auch gesundheitsgefährdend sein und/oder sind daher kennzeichnungspflichtig.

Die Anzahl an einsetzbaren Reaktivverdünner ist zudem gering, da es derzeit nur wenige verfügbare Reaktivverdünner auf dem Markt gibt. Die verfügbaren Reaktivverdünner tragen neben den radikalisch härtbaren funktionellen Gruppen keine oder nur eine sehr eingeschränkte Auswahl an anderen funktionellen Gruppen und haben daher oft nur wenig Einfluss auf die Eigenschaft der ausgehärteten Befestigungsmasse. Dies führt dazu, dass die Befestigungsmassen meist für bestimmte Anwendungen entwickelt werden, wie etwa bestimmte Temperaturbereiche, oder zur Anwendung in bestimmten Untergründen. Dies spricht für einen immensen Entwicklungsaufwand, um neue und breitere Anwendungen mit den Befestigungsmassen adressieren zu können.

Bisher werden Spezialprodukte, deren Formulierungen auf die speziellen Anwendungstemperaturen angepasst sind, hergestellt. Es gibt zwar Produkte, die für einen breiten Temperaturbereich gedacht sind, die jedoch über den gesamten Bereich gleiche Eigenschaften aufweisen. Gerade in den Randbereichen, d.h. bei niedrigen und bei hohen Temperaturen, muss entweder mit Beeinträchtigungen bei der Verarbeitbarkeit, bei der Aushärtung der Masse, oder bei den Eigenschaften der ausgehärteten Masse gerechnet werden. Es ist keine Befestigungsmasse bekannt, die einen sehr großen Temperaturbereich abdeckt, ohne Einbußen in den Randbereichen hinnehmen zu müssen.

Es besteht daher ein Bedarf an Befestigungsmassen deren Eigenschaften nicht durch den Einsatz von Reaktivverdünnern, sondern durch den Harzbestandteil beeinflusst werden kann.

Eine Aufgabe der vorliegenden Erfindung ist die Beeinflussung der Eigenschaften eines Reaktivharz-Masterbatches sowie eines daraus hergestellten Reaktivharzes, die allein auf die Struktur des Backbone-Harz zurückzuführen ist, nicht jedoch auf die Präsenz von zusätzlichen Verbindungen, wie z. B. Reaktivverdünner oder Additive. Hauptsächlich ist es Aufgabe der vorliegenden Erfindung die Eigenschaften eines Zwei- oder Mehrkomponenten-Reaktivharz-Systems mittels des enthaltenden Backbone-Harzes zu steuern. Insbesondere ist es Aufgabe der vorliegenden Erfindung Befestigungsmassen, wie beispielsweise Zwei- oder Mehrkomponenten-Reaktivharz-Systeme, bereitzustellen, deren Viskosität weniger von der Anwendungstemperatur der Befestigungsmasse abhängt, die insbesondere bei niedrigen Temperaturen, wie etwa unter 20°C, eine niedrige Viskosität aufweisen und damit die Bereitstellung von Reaktivharz-Systemen ermöglichen, die bei Anwendungstemperaturen unter 20°C, insbesondere bei Anwendungstemperaturen unter 10°C niedrigere Auspresskräfte aufweisen und damit anwenderfreundlicher sind als die herkömmlichen Befestigungssysteme.

Eine weitere Aufgabe der Erfindung ist die Bereitstellung einer Befestigungsmasse, welche niedrigere Auspresskräfte der Reaktivharz-Komponente aufweist als herkömmliche Massen.

Eine noch weitere Aufgabe der vorliegenden Erfindung ist die Bereitstellung einer Befestigungsmasse, welche hochgradig gesundheitsgefährdende Inhaltsstoffe in der Reaktivharz-Komponente vermeidet und gegebenenfalls auch kennzeichnungsfrei ist. Insbesondere ist es Aufgabe, den Anteil an Reaktivverdünnern in Reaktivharzen für die chemische Befestigung zu verringern, ohne auf deren Funktion bzw. Funktionen und positiven Auswirkungen auf die ausgehärtete Befestigungsmasse verzichten zu müssen.

Eine noch weitere Aufgabe der vorliegenden Erfindung ist die Bereitstellung einer Befestigungsmasse, welche sich durch gute Verarbeitbarkeit, Aushärteverhalten und durch geringen Schrumpf über eine große Temperaturspanne auszeichnet.

Diese Aufgaben werden durch die Verwendungen nach Anspruch 1, 7 und 8, ein diese Verbindungen enthaltendes Reaktivharz nach Anspruch 9, eine Reaktivharzkomponente nach Anspruch 10 und ein Reaktivharz-System nach Anspruch 12 gelöst.

Überraschender Weise wurde gefunden, dass durch die Verwendung bestimmter niedrigviskoser Epoxymethacrylat-Verbindungen als Backbone-Harz die Temperaturspanne, in der die Viskosität eines diese Verbindungen enthaltenden Reaktivharzes und einer daraus erhältlichen Reaktivharzkomponente von den Temperaturen weitestgehend wenig beeinflusst bleibt, groß ist.

Überraschend war, dass die Verbindungen trotz ihres relativ hohen Molekulargewichts eine niedrige Viskosität haben.

Vorteilhafterweise ermöglicht die vorliegende Erfindung bei niedrigen Anwendungstemperaturen niedrige Auspresskräfte bei einem Reaktivharz-System im Vergleich zu den herkömmlichen Systemen. Durch den Einsatz von niedrigviskosen Epoxymethacrylat-Verbindungen als Backbone-Harz in Reaktivharzen ist es somit gelungen, die Auspresskräfte eines Reaktivharz-Systems sowohl bei 20°C als auch bei niedrigeren Temperaturen, beispielsweise bei Temperaturen unter 10°C, bevorzugt unter 5°C zu senken, ohne dass hierzu ein hoher Anteil an Reaktivverdünner erforderlich ist.

Ferner wurde gefunden, dass es möglich ist, durch den Einsatz bestimmter niedrigviskoser Epoxymethacrylat-Verbindungen den Anteil an Reaktivverdünnern in Reaktivharzen für die chemische Befestigung zu verringern, ohne auf deren Funktion bzw. Funktionen und positiven Auswirkungen auf die ausgehärtete Befestigungsmasse verzichten zu müssen, da der Anteil an Backbone-Harz erhöht werden kann. Hierdurch wird es zum einen möglich, die Lastwerte einer ausgehärteten Masse zu steigern.

Die Erfindung beruht auf der Erkenntnis, dass es möglich ist, die bisher in Befestigungsmassen eingesetzten höherviskosen Harze durch kleinere, niedrigviskose Backbone-Harze zu ersetzen, um den Anteil an Reaktivverdünner zu verringern, ohne auf deren Funktionalität verzichten zu müssen.

Zum besseren Verständnis der Erfindung werden die folgenden Erläuterungen zum Herstellungsverfahren für ein Reaktivharz und zur hierin verwendeten Terminologie als sinnvoll erachtet.

Das Herstellungsverfahren für ein Reaktivharz verläuft, hier erläutert am Beispiel eines 1,4-Butandiol-diglycidylethers und Methacrylsäure, typischerweise wie folgt:

### 1. Herstellung Backbone-Harz/Reaktivharz-Masterbatch

1,4-Butandiol-diglycidylether und Methacrylsäure werden in Anwesenheit eines Katalysators und eines Inhibitors (dient dazu, das durch die Polymerisation gebildete Backbone-Harz zu stabilisieren, häufig auch Stabilisator oder Prozessstabilisator genannt) zur Reaktion gebracht. Hierbei entsteht das Backbone-Harz.

Das nach Ende der Reaktion erhaltene Reaktionsgemisch wird als Reaktivharz-Masterbatch bezeichnet. Dieses wird nicht weiter aufgearbeitet, d.h. das Backbone-Harz wird nicht isoliert.

### 2. Herstellung Reaktivharz

Zu dem Reaktivharz-Masterbatch werden nach Abschluss der Reaktion zum Backbone-Harz ein Beschleuniger-Inhibitor-System, d.h. eine Kombination aus einem oder mehreren zusätzlichen Inhibitoren und einem oder mehreren Beschleunigern sowie gegebenenfalls ein Reaktivverdünner gegeben.

Hierdurch erhält man das Reaktivharz.

Das Beschleuniger-Inhibitor-System dient dazu, die Reaktivität des Reaktiv-Harzes einzustellen, also den Zeitpunkt einzustellen, bis zu dem das Reaktiv-Harz nach Zugabe eines Initiators noch nicht vollständig ausgehärtet ist und bis zu welchem Zeitpunkt daher eine mit dem Reaktivharz angemischte Dübelmasse nach dem Mischen mit dem Initiator verarbeitbar bleibt.

Der Inhibitor in dem Beschleuniger-Inhibitor-System kann gleich dem Inhibitor bei der Herstellung des Backbone-Harzes sein, wenn dieser auch dazu geeignet ist, die Reaktivität einzustellen, oder ein anderer Inhibitor sein, wenn er nicht beide Funktionen aufweist. 4-Hydroxy-2,2,6,6-tetramethyl-piperidinyl-1-oxyl (TEMPOL) etwa kann als Stabilisator und als Inhibitor zur Einstellung der Reaktivität eingesetzt werden.

### 3. Herstellung Reaktivharzkomponente

Für die Verwendung des Reaktivharzes für bauliche Zwecke, insbesondere für die chemische Befestigung, werden nach der Herstellung des Reaktivharzes ein oder mehrere anorganische Zuschlagstoffe, wie Additive und/oder Füllstoffe, gegeben.

Hierdurch wird die Reaktivharzkomponente erhalten.

Im Sinne der Erfindung bedeuten die verwendeten Begriffe:
- "*Backbone-Harz"* ein üblicherweise festes oder hochviskoses radikalisch polymerisierbares Harz, welches durch Polymerisation (z.B. nach Zugabe eines Initiators in Gegenwart eines Beschleunigers) härtet und in der Regel ohne Reaktivverdünner und ohne weitere Reinigung vorliegt und damit Verunreinigungen enthalten kann;
- *"Reaktivharz-Masterbatch"* das Reaktionsprodukt der Reaktion zur Herstellung des Backbone-Harzes, also eine Mischung aus Backbone-Harz, Reaktivverdünner und gegebenenfalls weiteren Bestandteilen der Reaktionsmischung;
- "*Reaktivharz*" eine Mischung aus Reaktivharz-Masterbatch, mindestens einem Beschleuniger und mindestens einem Inhibitor (auch als Beschleuniger-Inhibitor-System bezeichnet), mindestens einem Reaktivverdünner und gegebenenfalls weiteren Additiven; das Reaktivharz ist typischerweise flüssig oder viskos und kann zu einer Reaktivharzkomponente weiterverarbeitet werden; hierin wird das Reaktivharz auch als "*Harzmischung*" bezeichnet;
- *"Inhibitor"* einen Stoff, der eine unerwünschte radikalische Polymerisation während der Synthese oder der Lagerung eines Harzes oder einer Harz-haltigen Zusammensetzung unterdrückt (diese Stoffe werden in Fachkreisen auch als *"Stabilisator"* bezeichnet) bzw. der eine radikalische Polymerisation eines Harzes nach Zugabe eines Initiators (üblicherweise in Verbindung mit einem Beschleuniger) zeitlich verzögert (diese Stoffe werden in Fachkreisen auch als *"Inhibitor"* bezeichnet - die jeweilige Bedeutung des Begriffes erschließt sich aus dem Kontext);
- *"Beschleuniger"* ein Reagenz, welches mit dem Initiator eine Reaktion eingeht, so dass bereits bei niedrigen Temperaturen durch den Initiator größere Mengen an Radikalen erzeugt werden, oder welches die Zerfallsreaktion des Initiators katalysiert;
- *"Reaktivverdünner"* flüssige oder niedrigviskose Monomere und Backbone-Harze, welche andere Backbone-Harze oder den Reaktivharz-Masterbatch verdünnen und dadurch die zu deren Applikation notwendige Viskosität verleihen, zur Reaktion mit dem Backbone-Harz befähigte funktionelle Gruppen enthalten und bei der Polymerisation (Härtung) zum überwiegenden Teil Bestandteil der gehärteten Masse (z.B. des Mörtels) werden; Reaktivverdünner werden auch co-polymerisierbares Monomer genannt;
- "*Reaktivharzkomponente*" eine flüssige oder viskose Mischung aus Reaktivharz und Füllstoffen und optional weiteren Komponenten, z.B. Additiven; typischerweise ist die Reaktivharzkomponente eine der beiden Komponenten eines Zweikomponenten-Reaktivharz-Systems zur chemischen Befestigung;
- *"Initiator"* einen Stoff, der (üblicherweise in Kombination mit einem Beschleuniger) reaktionsinitierende Radikale bildet;
- *"Härterkomponente"* eine Zusammensetzung, die einen Initiator für die Polymerisation eines Backbone-Harzes enthält; die Härterkomponente kann fest oder flüssig sein und neben dem Initiator ein Lösungsmittel sowie Füllstoffe und/oder Additive enthalten; typischerweise ist die Härterkomponente neben der Reaktivharzkomponente die andere der beiden Komponenten eines Zweikomponenten-Reaktivharz-Systems zur chemischen Befestigung;
- "*Mörtelmasse*/*Befestigungsmasse*" die Zusammensetzung, die durch das Mischen der Reaktivharzkomponente mit der Härterkomponente erhalten wird und als solche direkt zur chemischen Befestigung verwendet werden kann;
- "*Reaktivharz-System*" allgemein ein System, das voneinander getrennt gelagerte Komponenten umfasst, so dass eine Härtung des in einer Komponente enthaltenen Backbone-Harzes erst nach dem Mischen der Komponenten erfolgt;
- "*Zweikomponenten-System*" bzw. "*Zweikomponenten-Reaktivharz-System*" ein Reaktivharz-System, das zwei voneinander getrennt gelagerte Komponenten, eine Reaktivharzkomponente (A) und eine Härterkomponente (B), umfasst, so dass eine Härtung des in der Reaktivharzkomponente enthaltenen Backbone-Harzes erst nach dem Mischen der beiden Komponenten erfolgt;
- "*Mehrkomponenten-System*" bzw. "*Mehrkomponenten-Reaktivharz-System*" ein Reaktivharz-System, das mehrere voneinander getrennt gelagerte Komponenten umfasst, unter anderem eine Reaktivharzkomponente (A) und eine Härterkomponente (B), so dass eine Härtung des in der Reaktivharzkomponente enthaltenen Backbone-Harzes erst nach dem Mischen aller Komponenten erfolgt;
- *"bauliche Zwecke"* jegliche Anwendung zur Erstellung und Instandhaltung bzw. - setzung von Bauteilen und Bauwerken, als Polymerbeton, als Beschichtungsmasse auf Kunstharzbasis oder als kalthärtende Straßenmarkierung; insbesondere die Verstärkung von Bauteilen und Bauwerken, beispielsweise Wände, Decken oder Böden, die Befestigung von Bauteilen, wie Platten oder Blöcken, z. B. aus Stein, Glas oder Kunststoff, an Bauteilen oder Bauwerken, etwa durch Kleben (bauliches Kleben) und ganz besonders die chemische Befestigung von Verankerungsmitteln, wie Ankerstangen, Bolzen oder dergleichen in Aussparungen, wie Bohrlöchern;
- *"chemische Befestigung"* eine (stoff- und/oder formschlüssige) Befestigung von Verankerungsmitteln, wie Ankerstangen, Bolzen, Bewehrungseisen, Schrauben oder dergleichen in Aussparungen, wie Bohrlöchern, insbesondere in Bohrlöchern in verschiedenen Untergründen, insbesondere mineralischen Untergründen, wie solche auf der Grundlage von Beton, Porenbeton, Ziegelwerk, Kalksandstein, Sandstein, Naturstein, Glas und dergleichen, und metallischen Untergründen, wie solche aus Stahl;
- *"aliphatische Kohlenwasserstoffgruppe"* eine acyclische und cyclische, gesättigte oder ungesättigte Kohlenwasserstoffgruppe, die nicht aromatisch sind (PAC, 1995, 67, 1307; Glossary of class names of organic compounds and reactivity intermediates based on structure (IUPAC Recommendations 1995));
- *"(Meth)acryl...*/*..(meth)acryl...",* dass sowohl die "Methacryl.../...methacryl..."- als auch die "Acryl.../...acryl..."-Verbindungen gemeint sein sollen; bevorzugt sind in der vorliegenden Erfindung "Methacryl.../...methacryl... "-Verbindungen gemeint;
- *"ein", "eine", "einer"* als Artikel vor einer chemischen Verbindungsklasse, z.B. vor dem Wort "Epoxymethacrylat", dass mindestens eine, d.h., eine oder mehrere unter diese chemische Verbindungsklasse fallende Verbindungen, z.B. verschiedene Epoxymethacrylate, gemeint sein können. In einer bevorzugten Ausführungsform ist mit diesem Artikel nur eine einzelne Verbindung gemeint;
- *"mindestens ein", "mindestens eine", "mindestens einer"* zahlenmäßig *"ein oder mehrere".* In einer bevorzugten Ausführungsform ist mit diesem Begriff zahlenmäßig *"ein", "eine", "einer"* gemeint;
- *"enthalten" und "umfassen",* dass neben den genannten Bestandteilen noch weitere vorhanden sein können. Diese Begriffe sind einschließlich gemeint und umfassen daher auch *"bestehen aus". "Bestehen aus"* ist abschließend gemeint und bedeutet, dass keine weiteren Bestandteile vorhanden sein können. In einer bevorzugten Ausführungsform bedeuten die Begriffe *"enthalten" und "umfassen"* den Begriff *"bestehen aus",*
- *"etwa"* oder *"circa"* vor einem Zahlenwert einen Bereich von ± 5% dieses Wertes, bevorzugt ± 2% dieses Wertes, bevorzugter ± 1% dieses Wertes, besonders bevorzugt ± 0% dieses Wertes (also genau diesen Wert);
- ein durch Zahlen begrenzter Bereich, dass die beiden Eckwerte und jeder Wert innerhalb dieses Bereichs einzeln offenbart sind.

Alle in diesem Text genannten Normen (z.B. DIN-Normen) wurden in der zum Anmeldetag dieser Anmeldung aktuellen Ausgabe verwendet.

Ein erster Gegenstand der Erfindung ist die Verwendung einer Verbindung der allgemeinen Formel (I) 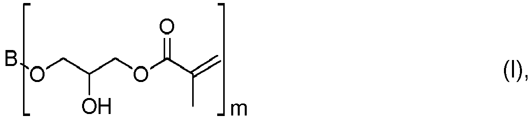 worin
m eine ganze Zahl größer oder gleich 2 ist, und
B eine lineare, verzweigte oder cyclische aliphatische Kohlenwasserstoffgruppe ist in einem Reaktivharz oder einer Reaktivharzkomponente für die chemische Befestigung von Verankerungsmitteln in Aussparungen

Ein zweiter Gegenstand ist deren Verwendung zur Erniedrigung der Viskosität eines Reaktivharzes oder einer Reaktivharzkomponente für bauliche Zwecke. Ein dritter Gegenstand ist deren Verwendung zur Erniedrigung der Auspresskräfte einer Reaktivharzkomponente oder eines Reaktivharz-Systems. Ein vierter Gegenstand ist ein Reaktivharz umfassend die Verbindung der allgemeinen Formel (I), einen Inhibitor, einen Beschleuniger und gegebenenfalls einen Reaktivverdünner. Ein fünfter Gegenstand ist die Verwendung des Reaktivharzes für bauliche Zwecke, wie die chemische Befestigung von Verankerungsmitteln in Bohrlöchern.

Erfindungsgemäß wird als niedrigviskose Epoxymethacrylat-Verbindung eine Verbindung der allgemeinen Formel (I) 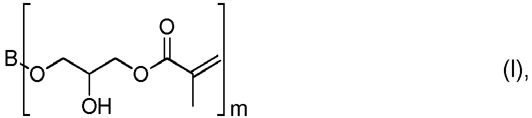 worin
m eine ganze Zahl größer oder gleich 2, ist und
B eine lineare, verzweigte oder cyclische aliphatische Kohlenwasserstoffgruppe ist
verwendet.

Die aliphatische Kohlenwasserstoffgruppe B kann substituiert sein, insbesondere hydroxysubstituiert sein. Vorzugsweise ist die aliphatische Kohlenwasserstoffgruppe eine lineare oder verzweigte Alkylengruppe, die gegebenenfalls hydroxysubstituiert ist. Die Alkylengruppe ist vorzugsweise eine C₂-C₁₂-Alkylengruppe, bevorzugt eine C₂-C₈-Alkylengruppe, noch stärker bevorzugt eine C₂-C₆-Alkylengruppe. Die Alkylengruppe kann substituiert sein, insbesondere durch einen Alkylrest.

Geeignete Alkylengruppen sind n-wertige Gruppen, wie sie durch Entfernung der Glycidylgruppen aus einer aliphatischen Polyglycidylverbindung erhalten werden, wobei n für die Anzahl der Glycidylgruppen (Wertigkeit) in der Polyglycidylverbindung steht. m entspricht dabei der Anzahl der Glycidylgruppen in der Polyglycidylverbindung und damit der Wertigkeit der Polyglycidylverbindung. Die Polyglycidylverbindung sind von Polyalkoholen abgeleitet, bei denen die Hydroxylgruppen ganz oder teilweise durch Umsetzen mit Epihalogenhydrin in eine Glycidylethergruppe umgewandelt werden. Geeignete Polyalkohole sind beispielsweise 1,2-Ethandiol, 1,2-Propandiol, 1,3-Propandiol, 1,3-Butandiol, 1,4-Butandiol, 1,6-Hexandiol, Glycerin, Neopentylglykol, Ethylenglykol, Cyclohexandimethanol, Trimethylolpropan, Pentaerythrit und Polyethylenglykole.
m ist bevorzugt eine ganze Zahl zwischen 2 und 5, stärker bevorzugt zwischen 2 und 4, noch stärker bevorzugt 2 oder 3.

Ist m gleich 2, so weist die Verbindung der Formel 1 zwei endständige Methacrylatgruppen auf, die radikalisch polymerisieren können.

Ist m größer als 2, so weist die Verbindung der Formel 1 mehr als zwei endständige Methacrylatgruppen auf, die radikalisch polymerisieren können. Durch die zusätzliche Methacrylatgruppe, etwa einer dritten Methacrylatgruppe wenn m gleich 3 ist, wird eine Verzweigungsstelle bereitgestellt, so dass ein höherer Verzweigungsgrad erreicht werden kann. Hierdurch wird die Möglichkeit der höheren Quervernetzung geschaffen. Es wird erwartet, dass sich ein höher verzweigtes Polymernetzwerk bildet. Dies ist besonders dann vorteilhaft, wenn auf tri- oder multi-funktionelle Reaktivverdünner verzichtet aber die Möglichkeit der Quervernetzung beibehalten werden soll.

Bevorzugte Verbindungen mit zwei Methacrylatgruppen haben die Struktur (II), (III), (IV) und (V):

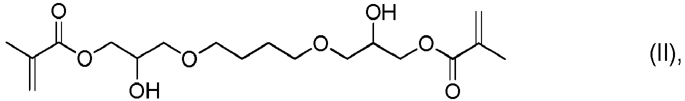

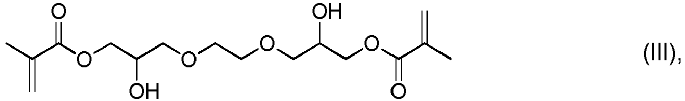

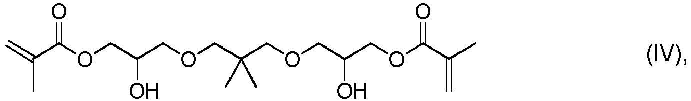

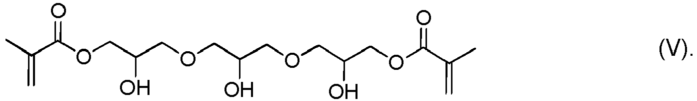

Eine bevorzugte Verbindung mit drei Methacrylatgruppen hat die Struktur (VI):

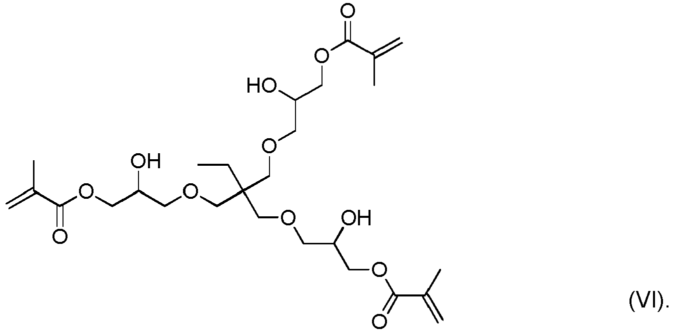

Die niedrigviskosen Epoxymethacrylat-Verbindungen können durch Umsetzen von Methacrylsäure mit einem multifunktionellen Glycidylether erhalten werden. Zweckmäßig werden pro Epoxidäquivalent 0,7 bis 1,2 Carboxyläquivalente Methacrylsäure eingesetzt. Die Epoxidgruppen aufweisenden organischen Verbindungen und die Methacrylsäure werden dabei bevorzugt in etwa stöchiometrischen Verhältnissen eingesetzt, d.h. pro Epoxidäquivalent der organischen Verbindung wird etwa ein Äquivalent Methacrylsäure eingesetzt. Der Gylcidylether und die Methacrylsäure werden in Anwesenheit eines Katalysators und gegebenenfalls eines Inhibitors, welcher dazu dient, das gebildete Backbone-Harz zu stabilisieren zur Reaktion gebracht. Hierbei entsteht das Backbone-Harz.

Geeignete Glycidylether sind aliphatische oder alicyclische Glycidylether aus Polyolen (mehrwertige Alkohole) mit einer Epoxidfunktionalität von mindestens 2, wie beispielsweise 1,4-Butandioldiglycidylether (BDDGE), Cyclohexandimethanoldiglycidylether, Hexandioldiglycidylether und/oder insbesondere Tri- oder höhere Glycidylether, z.B. Glycerintriglycidylether, Pentaerythrittetraglycidylether oder Trimethylolpropantriglycidylether (TMPTGE).

Darüber hinaus können niedrigviskose Glycidylether verwendet werden, die in EpoxyAmin-Systemen als Reaktivverdünner eingesetzt werden können und die eine Epoxydfunktionalität von mindestens zwei haben.

Die Verbindungen der allgemeinen Formel (I) werden erfindungsgemäß einzeln oder als Gemisch von mindestens zwei Verbindungen als Backbone-Harz in Reaktivharz-Zusammensetzungen, wie Reaktivharzen und Reaktivharzkomponenten, eingesetzt. Werden mehrere Verbindungen der allgemeinen Formel (I) als Backbone-Harz eingesetzt, kann über die Auswahl der Verbindungen und den Anteil an Verbindungen, in denen m größer als 2 ist, die Struktur des gebildeten Polymernetzwerks beeinflusst werden.

Die erfindungsgemäß verwendeten Verbindungen können dabei allein oder zusätzlich zu anderen für den jeweiligen Anwendungszweck des Reaktivharzes üblicherweise verwendeten Harze verwendet werden. Damit kann gegebenenfalls die Vernetzungsdichte des Polymernetzwerks beeinflusst werden.

Für den Fall, dass bei der Herstellung der erfindungsgemäß verwendeten Verbindungen nicht alle Glycidylgruppen umgesetzt werden oder ein Teil der Glycidylgruppen vor der Reaktion etwa durch eine Nebenreaktion geöffnet werden, werden Verbindungen erhalten, die entweder als Hauptverbindungen oder als Verunreinigungen in den Reaktionsharz-Masterbatches enthalten sein können.

Die Verbindungen der allgemeinen Formel (I) werden erfindungsgemäß zur Herstellung eines Reaktivharzes verwendet. Hierdurch kann die Viskosität des so hergestellten Reaktivharzes verringert werden, ohne dass ein hoher Anteil an Reaktivverdünnern erforderlich wird, wie dies bei den handelsüblichen Massen der Fall ist und ohne dass die mit einem hohen Anteil an Reaktivverdünnern einhergehenden Probleme, wie etwa die Verringerung der erreichbaren Lastwerte der ausgehärteten Masse auftreten. Damit kann eine Erniedrigung der Auspresskräfte eines Reaktivharz-Systems, welches erfindungsgemäß die oben beschriebenen Verbindungen enthält, erreicht werden.

Das erfindungsgemäße Reaktivharz enthält eine Verbindung der allgemeinen Formel (I) wie oben beschrieben als Backbone-Harz, einen Inhibitor, einen Beschleuniger und gegebenenfalls einen Reaktivverdünner. Da das Backbone-Harz nach seiner Herstellung typischerweise ohne Isolierung für die Herstellung des Reaktivharzes verwendet wird, sind in der Regel auch noch die neben dem Backbone-Harz im Reaktivharz-Masterbatch enthaltenen weiteren Bestandteile im Reaktivharz vorhanden, wie etwa einen Katalysator.

Der Anteil der Verbindung der allgemeinen Formel (I) in dem erfindungsgemäßen Reaktivharz beträgt von 25 Gew.-% bis 65 Gew.-%, vorzugsweise von 30 Gew.-% bis 45 Gew.-%, besonders bevorzugt von 35 Gew.-% bis 40 Gew.-%, ganz besonders bevorzugt von 33 Gew.-% bis 40 Gew.-% bezogen auf das Gesamtgewicht des Reaktivharzes.

Als Inhibitor sind die für radikalisch polymerisierbare Verbindungen üblicherweise verwendeten stabilen Radikale, wie *N*-Oxyl-Radikale geeignet, wie sie dem Fachmann bekannt sind.

Der Inhibitor kann zum einen dazu dienen, eine unerwünschte radikalische Polymerisation während der Synthese des Backbone-Harzes oder der Lagerung des Reaktivharzes und der Reaktivharzkomponente zu unterdrücken. Er kann auch dazu dienen - gegebenenfalls zusätzlich - die radikalische Polymerisation des Backbone-Harzes nach Zugabe des Initiators zeitlich zu verzögern und dadurch die Verarbeitungszeit des Reaktivharzes oder der Reaktivharzkomponente nach dem Mischen mit dem Härtungsmittel einzustellen.

Als stabile *N*-Oxyl-Radikale können beispielsweise solche verwendet werden, wie sie in der DE 199 56 509 A1 und der DE 195 31 649 A1 beschrieben sind. Derartige stabile Nitroxylradikale sind vom Typ Piperidinyl-*N*-oxyl oder Tetrahydropyrrol-*N*-oxyl oder eine Mischung daraus.

Bevorzugte stabile Nitroxylradikale sind ausgewählt aus der Gruppe bestehend aus 1-Oxyl-2,2,6,6-tetramethylpiperidin, 1-Oxyl-2,2,6,6-tetramethylpiperidin-4-ol (ebenso als TEMPOL bezeichnet), 1-Oxyl-2,2,6,6-tetramethylpiperidin-4-on (ebenso als TEMPON bezeichnet), 1-Oxyl-2,2,6,6-tetramethyl-4-carboxyl-piperidin (ebenso als 4-Carboxy-TEMPO bezeichnet), 1-Oxyl-2,2,5,5-tetramethylpyrrolidin, 1-Oxyl-2,2,5,5-tetramethyl-3-carboxylpyrrolidin (ebenso als 3-Carboxy-PROXYL bezeichnet) und Mischungen aus zwei oder mehreren dieser Verbindungen, wobei 1-Oxyl-2,2,6,6-tetramethylpiperidin-4-ol (TEMPOL) besonders bevorzugt ist.

Neben dem Nitroxylradikal vom Typ Piperidinyl-*N*-oxyl oder Tetrahydropyrrol-*N*-oxyl können sowohl zur weiteren Stabilisierung des Reaktivharzes oder der das Reaktivharz enthaltenden Reaktivharzkomponente (A) oder anderer das Reaktivharz enthaltender Zusammensetzungen als auch zur Einstellung der Harzreaktivität ein oder mehrere weitere Inhibitoren vorhanden sein.

Hierfür sind die für radikalisch polymerisierbare Verbindungen üblicherweise verwendeten Inhibitoren geeignet, wie sie dem Fachmann bekannt sind. Bevorzugt sind diese weiteren Inhibitoren unter phenolischen Verbindungen und nicht-phenolischen Verbindungen und/oder Phenothiazinen, ausgewählt.

Als phenolische Inhibitoren, sind Phenole, wie 2-Methoxyphenol, 4-Methoxyphenol, 2,6-Di-tert-butyl-4-methylphenol, 2,4-Di-tert-butylphenol, 2,6-Di-tert-butylphenol, 2,4,6-Trimethylphenol, 2,4,6-Tris(dimethylaminomethyl)phenol, 4,4'-Thio-bis(3-methyl-6-tert-butylphenol), 4,4'-Isopropylidendiphenol, 6,6'-Di-tert-butyl-4,4'-bis(2,6-di-tert-butylphenol), 1,3,5-Trimethyl-2,4,6-tris(3,5-di-tert-butyl-4-hydroxybenzyl)benzol, 2,2'-Methylen-di-p-cresol, Catechole, wie Brenzkatechin, und Catecholderivate, wie Butylbrenzkatechine, wie 4-tert-Butylbrenzkatechin und 4,6-Di-tert-butylbrenzkatechin, Hydrochinone, wie Hydrochinon, 2-Methylhydrochinon, 2-tert-Butylhydrochinon, 2,5-Di-tert-butylhydrochinon, 2,6-Di-tert-butylhydrochinon, 2,6-Dimethylhydrochinon, 2,3,5-Trimethylhydrochinon, Benzochinon, 2,3,5,6-Tetrachloro-1,4-benzochinon, Methylbenzochinon, 2,6-Dimethylbenzochinon, Naphthochinon, oder Gemische von zweien oder mehreren davon, geeignet. Diese Inhibitoren sind oft Bestandteil von kommerziellen radikalisch härtenden Reaktivharzkomponenten.

Als nicht-phenolische Inhibitoren kommen vorzugsweise Phenothiazine, wie Phenothiazin und/oder Derivate oder Kombinationen davon, oder stabile organische Radikale, wie etwa Galvinoxyl- und *N*-oxyl-Radikale, jedoch nicht vom Piperidinyl-*N-*oxyl- oder Tetrahydropyrrol-*N*-oxyl-Typ, in Betracht, wie Aluminium-*N-*nitrosophenylhydroxylamin, Diethylhydroxylamin, Oxime, wie Acetaldoxim, Acetonoxim, Methylethylketoxim, Salicyloxim, Benzoxim, Glyoxime, Dimethylglyoxim, Aceton-O-(benzyloxycarbonyl)oxim und dergleichen.

Ferner können in para-Stellung zur Hydroxylgruppe substituierte Pyrimidinol- oder Pyridinol-Verbindungen, wie sie in der Patentschrift DE 10 2011 077 248 B1 beschrieben sind, als Inhibitoren eingesetzt werden.

Bevorzugt sind die weiteren Inhibitoren ausgewählt aus der Gruppe der Catechole, Catecholderivate, Phenothiazine, tert-Butylcatechol, Tempol oder ein Gemisch von zwei oder mehr davon. Besonders bevorzugt sind die weiteren Inhibitoren ausgewählt aus der Gruppe der Catechole und Phenothiazine. Ganz besonders bevorzugt sind die in den Beispielen verwendeten weiteren Inhibitoren, bevorzugt etwa in den in den Beispielen angegebenen Mengen.

Die weiteren Inhibitoren können, abhängig von den gewünschten Eigenschaften des Reaktivharzes, entweder alleine oder als Kombination von zweien oder mehreren davon verwendet werden.

Der Inhibitor oder die Inhibitormischung wird zugesetzt in der Fachwelt bekannten üblichen Mengen, bevorzugt in einer Menge von etwa 0,0005 bis etwa 2 Gew.-% (bezogen auf das - letztendlich damit hergestellte - Reaktivharz), stärker bevorzugt von etwa 0,01 bis etwa 1 Gew.-% (bezogen auf das Reaktivharz), noch stärker bevorzugt von etwa 0,05 bis etwa 1 Gew.-% (bezogen auf das Reaktivharz), noch stärker bevorzugt von etwa 0,2 bis etwa 0,5 Gew.-% (bezogen auf das Reaktivharz).

Die Verbindungen der allgemeinen Formel (I), insbesondere bei der Verwendung in Reaktivharzen und Reaktivharzkomponenten für die chemische Befestigung und das bauliche Kleben, werden im Allgemeinen durch Peroxide als Härtungsmittel gehärtet. Die Peroxide werden bevorzugt durch einen Beschleuniger initiiert, damit auch bei niedrigen Anwendungstemperaturen eine Polymerisation stattfindet. Der Beschleuniger wird bereits dem Reaktivharz zugegeben.

Geeignete Beschleuniger, die dem Fachmann bekannt sind, sind beispielsweise Amine, bevorzugt tertiäre Amine und/oder Metallsalze.

Geeignete Amine sind unter folgenden Verbindungen ausgewählt: Dimethylamin, Trimethylamin, Ethylamin, Diethylamin, Triethylamin, n-Propylamin, Di-n-propylamin, Tri-n-propylamin, Isopropylamin, Diisopropylamin, Triisopropylamin, n-Butylamin, Isobutylamin, tert-Butylamin, Di-n-butylamin, Diisobutylamin, Tri-isobutylamin, Pentylamin, Isopentylamin, Diisopentylamin, Hexylamin, Octylamin, Dodecylamin, Laurylamin, Stearylamin, Aminoethanol, Diethanolamin, Triethanolamin, Aminohexanol, Ethoxyaminoethan, Dimethyl-(2-chloroethyl)amin, 2-Ethylhexylamin, Bis-(2-chloroethyl)amin, 2-Ethylhexylamin, Bis-(2-ethylhexyl)amin, N-Methylstearylamin, Dialkylamine, Ethylendiamin, N,N'-Dimethylethylendiamin, Tetramethylethylendiamin, Diethylentriamin, Permethyldiethylentriamin, Triethylentetramin, Tetraethylenpentamin, 1,2-Diaminopropan, Di-propylentriamin, Tripropylentetramin, 1,4-Diaminobutan, 1,6-Diaminohexan, 4-Amino-1-diethylaminopentan, 2,5-Diamino-2,5-dimethylhexan, Trimethylhexamethylendiamin, N,N-Dimethylaminoethanol, 2-(2-Diethylaminoethoxy)ethanol, Bis-(2-hydroxyethyl)-oleylamin, Tris-[2-(2-hydroxy-ethoxy)-ethyl]amin, 3-Amino-1-propanol, Methyl-(3-aminopropyl)ether, Ethyl-(3-aminopropyl)ether, 1,4-Butandiol-bis(3-aminopropylether), 3-Dimethylamino-1-propanol, 1-Amino-2-propanol, 1-Diethylamino-2-propanol, Diisopropanolamin, Methyl-bis-(2-hydroxypropyl)-amin, Tris-(2-hydroxypropyl)amin, 4-Amino-2-butanol, 2-Amino-2-methylpropanol, 2-Amino-2-methyl-propandiol, 2-Amino-2-hydroxymethylpropandiol, 5-Aiethylamino-2-pentanon, 3-Methylaminopropionsäurenitril, 6-Aminohexansäure, 11-Aminoundecansäure, 6-Aminohexansäureethylester, 11-Aminohexansäureisopropylester, Cyclohexylamin, N-Methylcyclohexylamin, N,N-Dimethylcyclohexylamin, Dicyclohexylamin, N-Ethylcyclohexylamin, N-(2-Hydroxyethyl)-cyclohexylamin, N,N-Bis-(2-hydroxyethyl)-cyclohexylamin, N-(3-Aminopropyl)-cyclohexylamin, Aminomethylcyclohexan, Hexahydrotoluidin, Hexahydrobenzylamin, Anilin, N-Methylanilin, N,N-Dimethylanilin, N,N-Diethylanilin, N,N-Di-propylanilin, iso-Butylanilin, Toluidine, Diphenylamin, Hydroxyethylanilin, Bis-(hydroxyethyl)anilin, Chloranilin, Aminophenole, Aminobenzoesäuren und deren Ester, Benzylamin, Dibenzylamin, Tribenzylamin, Methyldibenzylamin, a-Phenylethylamin, Xylidin, Diisopropylanilin, Dodecylanilin, Aminonaphthalin, N-Methylaminonaphthalin, N,N-Dimethylaminonaphthalin, N,N-Dibenzylnaphthalin, Diaminocyclohexan, 4,4'-Diaminodicyclohexylmethan, Diamino-dimethyl-dicyclohexylmethan, Phenylendiamin, Xylylendiamin, Diaminobiphenyl, Naphthalindiamine, Toluidine, Benzidine, 2,2-Bis-(aminophenyl)-propan, Aminoanisole, Amino-thiophenole, Aminodiphenylether, Aminocresole, Morpholin, N-Methylmorpholin, N-Phenylmorpholin, Hydroxyethylmorpholin, N-Methylpyrrolidin, Pyrrolidin, Piperidin, Hydroxyethylpiperidin, Pyrrole, Pyridine, Chinoline, Indole, Indolenine, Carbazole, Pyrazole, Imidazole, Thiazole, Pyrimidine, Chinoxaline, Aminomorpholin, Dimorpholinethan, [2,2,2]-Diazabicyclooctan und N,N-Dimethyl-p-toluidin.

Als Beschleuniger wird erfindungsgemäß Di-iso-propanol-p-toluidin oder N,N-Bis(2-hydroxyethyl)-m-toluidin eingesetzt.

Bevorzugte Amine sind Anilin-Derivate und N,N-Bisalkylarylamine, wie N,N,-Dimethylanilin, N,N-Diethylanilin, N,N-Dimethyl-p-toluidin, N,N-Bis(hydroxyalkyl)arylamine, N,N-Bis(2-hydroxyethyl)aniline, N,N-Bis(2-hydroxyethyl)toluidin, N,N-Bis(2-hydroxypropyl)anilin, N,N-Bis(2-hydroxypropyl)toluidin, N,N-Bis(3-methacryloyl-2-hydroxypropyl)-p-toluidin, N,N-Dibutoxyhydroxypropyl-p-toluidin und 4,4'-Bis(dimethylamino)diphenylmethan. Besonders bevorzugt ist Di-iso-propanol-p-toluidin.

Polymere Amine, wie solche die durch Polykondensation von N,N-Bis(hydroxylalkyl)anilin mit Dicarbonsäuren oder durch Polyaddition von Ethylenoxid oder anderen Epoxiden und diese Amine erhalten werden, sind ebenso als Beschleuniger geeignet.

Geeignete Metallsalze sind, zum Beispiel Cobaltoctoat oder Cobaltnaphthenoat sowie Vanadium-, Kalium-, Kalzium-, Kupfer-, Mangan- oder Zirkoniumcarboxylate. Weitere geeignete Metallsalze sind die weiter oben beschriebenen Zinn-Katalysatoren.

Sofern ein Beschleuniger verwendet wird, wird er in einer Menge von 0,01 bis 10 Gew.-%, bevorzugt 0,2 bis 5 Gew.-%, bezogen auf das Reaktivharz, eingesetzt.

Das Reaktivharz kann noch einen Reaktivverdünner enthalten, sofern dies erforderlich wird.

Geeignete Reaktivverdünner sind niederviskose, radikalisch co-polymerisierbare Verbindungen, bevorzugt kennzeichnungsfreie Verbindungen, die zugegeben werden um unter anderem die Viskosität des Epoxymethacrylats bzw. der Vorstufen bei dessen Herstellung anzupassen, falls erforderlich.

Geeignete Reaktivverdünner sind in den Anmeldungen EP 1 935 860 A1 und DE 195 31 649 A1 beschrieben. Vorzugsweise enthält das Reaktivharz (die Harzmischung) als Reaktivverdünner einen (Meth)acrylsäureester, wobei besonders bevorzugt aliphatische oder aromatische C₅-C₁₅-(Meth)acrylate ausgewählt werden. Geeignete Beispiele umfassen: 2-Hydroxypropyl(meth)acrylat, 3-Hydroxypropyl(meth)acrylat, 1,2-Ethandioldi(meth)acrylat, 1,3-Propandioldimethacrylat, 1,3-Butandioldi(meth)acrylat, 1,4-Butandioldi(meth)acrylat, Trimethylolpropantri(meth)acrylat, Phenylethyl(meth)acrylat, Tetrahydrofurfuryl(meth)acrylat, Ethyltriglykol(meth)acrylat, /V,/V-Dimethylaminoethyl(meth)acrylat, N,N-Dimethylaminomethyl(meth)acrylat, N,N-Diethylaminoethyl(meth)acrylat, Acetoacetoxyethyl(meth)acrylat, Isobornyl(meth)acrylat, 2-Ethylhexyl(meth)acrylat, tert-Butylcyclohexyl(meth)acrylat, Benzyl(meth)acrylat, Methyl(meth)acrylat, n-Butyl(meth)acrylat, iso-Butyl(meth)acrylat, 3-Trimethoxysilylpropyl(meth)acrylat, Isodecyl(meth)acrylat, Diethylenglykoldi(meth)acrylat, Triethylenglykoldi(meth)acrylat, Methoxypolyethylenglykolmono(meth)acrylat, Trimethylcyclohexyl(meth)acrylat, 2-Hydroxyethyl(meth)acrylat, Dicyclopentenyloxyethyl(meth)acrylat und/oder Tricyclopentadienyldi(meth)acrylat, Bisphenol-A-(meth)acrylat, Novolakepoxidi(meth)acrylat, Di-[(meth)acryloyl-maleoyl]-tricyclo-5.2.1,0.2.6-decan, 3-(Meth)acryloyl-oxymethyl-tricylo-5.2.1.0.2.6-decan, 3-(Meth)cyclopentadienyl(meth)acrylat, und Decalyl-2-(meth)acrylat; PEG-di(meth)acrylat wie PEG200-di(meth)acrylat, Tetraehtylenglykoldi(meth)acrylat, Solketal(meth)acrylat, Cyclohexyl(meth)acrylat, Phenoxyethyldi(meth)acrylat, 2-Phenoxyethyl(meth)acrylat, Hexandiol-1,6-di(meth)acrylat, 1,2-Butandioldi(meth)acrylat, Methoxyethyl(meth)acrylat, Butyldiglykol(meth)acrylat, tert-Butyl(meth)acrylat und Norbornyl(meth)acrylat. Methacrylate sind gegenüber Acrylaten bevorzugt.

Besonders bevorzugt sind 2- und 3-Hydroxypropylmethacrylat, 1,2-Ethandioldimethacrylat, 1,4-Butandioldimethacrylat, 1,3-Butandioldimethacrylat, Glyceroldimethacrylat, Trimethylolpropantrimethacrylat, Acetoacetoxyethylmethacrylat, Isobornylmethacrylat, Bisphenol-A-dimethacrylat, ethoxylierte Bisphenol-A-methacrylate wie E2BADMA oder E3BADMA, Trimethylcyclohexylmethacrylat, 2-Hydroxyethylmethacrylat, PEG200-dimethacrylat und Norbornylmethacrylat und ganz besonders bevorzugt sind ein Gemisch aus 2- und 3-Hydroxypropylmethacrylat und 1,4-Butandioldimethacrylat oder eine Mischung aus diesen drei Methacrylaten.

Am bevorzugtesten ist ein Gemisch aus 2- und 3-Hydroxypropylmethacrylat. Grundsätzlich können auch andere übliche radikalisch polymerisierbare Verbindungen, allein oder im Gemisch mit den (Meth)acrylsäureestern, als Reaktivverdünner eingesetzt werden, z. B. Methacrylsäure, Styrol, α-Methylstyrol, alkylierte Styrole, wie tert-Butylstyrol, Divinylbenzol und Vinyl- sowie Allylverbindungen, wobei die nicht kennzeichnungspflichtigen Vertreter davon bevorzugt sind. Beispiele für derartige Vinyl- oder Allylverbindungen sind Hydroxybutylvinylether, Ethylenglycoldivinylether, 1,4-Butandioldivinylether, Trimethylolpropandivinylether, Trimethylolpropantrivinylether, Mono-, Di-, Tri-, Tetra- und Polyalkylenglycolvinylether, Mono-, Di-, Tri-, Tetra- und Polyalkylenglycolallylether, Adipinsäuredivinylester, Trimethylolpropandiallylether und Trimethylolpropantriallylether.

Der bzw. die Reaktivverdünner wird bzw. werden in einer Menge bis zu 65 Gew.-%, vorzugsweise bis zu 60 Gew.-%, weiter bevorzugt bis zu 55 Gew.-%, besonders bevorzugt in Mengen unter 50 Gew.-%, bezogen auf das Reaktivharz, zugegeben werden.

Ein beispielhaftes Reaktivharz umfasst eine Verbindung der allgemeinen Formel (I) 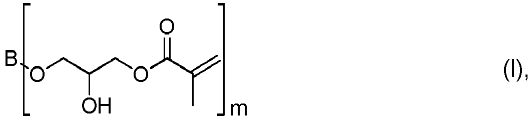 worin m eine ganze Zahl größer oder gleich 2 ist, und B eine lineare, verzweigte oder cyclische aliphatische Kohlenwasserstoffgruppe ist, als Backbone-Harz, ein stabiles Nitroxylradikal als Inhibitor, ein substituiertes Toluidin als Beschleuniger und gegebenenfalls einen Reaktivverdünner.

Ein bevorzugtes Reaktivharz umfasst eine Verbindung der allgemeinen Formel (I) 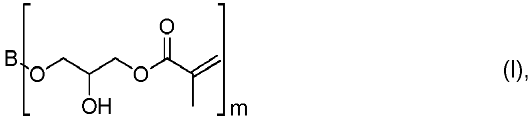 worin m eine ganze Zahl größer oder gleich 2 ist, und B eine lineare, verzweigte oder cyclische aliphatische Kohlenwasserstoffgruppe ist, als Backbone-Harz, ein stabiles Nitroxylradikal als Inhibitor, ein substituiertes Toluidin als Beschleuniger und gegebenenfalls einen Reaktivverdünner.

Ein weiter bevorzugtes Reaktivharz umfasst eine Verbindung der Formel (II), (III), (IV), (V) oder (VI) 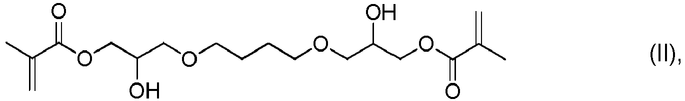 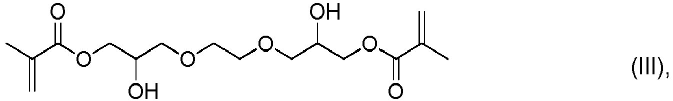 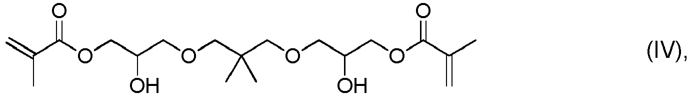 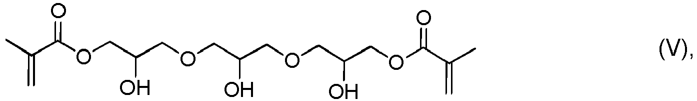 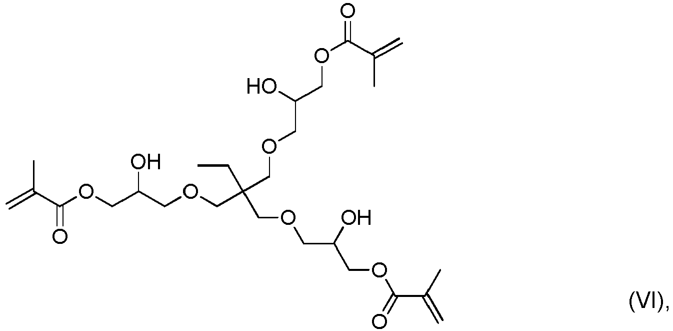 als Backbone-Harz, ein stabiles Nitroxylradikal als Inhibitor, ein substituiertes Toluidin als Beschleuniger und gegebenenfalls einen Reaktivverdünner.

Ein besonders bevorzugtes Reaktivharz umfasst eine Verbindung der Formel (II), (III), (IV), (V) oder (VI) als Backbone-Harz, 4-Hydroxy-2,2,6,6-tetramethyl-piperidinyl-1-oxyl (TEMPOL) als Inhibitor, Di-iso-propanol-p-toluidin als Beschleuniger und eine Mischung aus Hydroxypropylmethacrylat (HPMA) und 1,4-Butandioldimethacrylat (BDDMA) als Reaktivverdünner.

Ein erfindungsgemäßes Reaktivharz weist aufgrund des niedrigviskosen Backbone-Harzes eine besonders niedrige dynamische Viskosität auf, so dass es möglich wird, eine Reaktivharzkomponente für ein Reaktivharz-System herzustellen, welche bei Anwendungstemperaturen unter 10°C, vorzugsweise bei 0°C wesentlich niedrigere Auspresskräfte zeigt als bei den herkömmlichen Systemen, ohne die dafür bisher benötigten hohen Anteile an Reaktivverdünnern.

Eine Reaktivharzkomponente kann neben dem erfindungsgemäßen Reaktivharz anorganische Zuschlagstoffe, wie Füllstoffe und/oder Additive, enthalten. Es sei darauf hingewiesen, dass einige Stoffe sowohl als Füllstoff und, ggf. in modifizierter Form, auch als Additiv verwendet werden können. Beispielsweise dient pyrogene Kieselsäure in ihrer polaren, nicht nachbehandelten Form eher als Füllstoff und in ihrer apolaren, nachbehandelten Form eher als Additiv. In Fällen, in denen genau derselbe Stoff als Füllstoff oder Additiv verwendet werden kann, soll seine Gesamtmenge die hierin festgelegte Obergrenze für Füllstoffe nicht überschreiten. Zur Herstellung einer Reaktivharzkomponente für bauliche Zwecke, inbesondere die chemische Befestigung können dem erfindungsgemäßen Reaktivharz übliche Füllstoffe und/oder Additive zugegeben werden. Diese Füllstoffe sind typischerweise anorganische Füllstoffe und Additive, wie beispielsweise weiter unten beschrieben. Der Anteil des Reaktivharzes in der Reaktivharzkomponente beträgt bevorzugt von etwa 10 bis etwa 70 Gew.-%, stärker bevorzugt von etwa 30 bis etwa 50 Gew.-%, bezogen auf die Reaktivharzkomponente. Dementsprechend beträgt der Anteil der Füllstoffe bevorzugt von etwa 90 bis etwa 30 Gew.-%, stärker bevorzugt von etwa 70 bis etwa 50 Gew.-%, bezogen auf die Reaktivharzkomponente.

Als Füllstoffe finden übliche Füllstoffe, vorzugsweise mineralische oder mineralähnliche Füllstoffe, wie Quarz, Glas, Sand, Quarzsand, Quarzmehl, Porzellan, Korund, Keramik, Talkum, Kieselsäure (z.B. pyrogene Kieselsäure, insbesondere polare, nicht nachbehandelte pyrogene Kieselsäure), Silikate, Aluminiumoxide (z.B. Tonerde), Ton, Titandioxid, Kreide, Schwerspat, Feldspat, Basalt, Aluminiumhydroxid, Granit oder Sandstein, polymere Füllstoffe, wie Duroplaste, hydraulisch härtbare Füllstoffe, wie Gips, Branntkalk oder Zement (z.B. Aluminatzement (oft auch als Tonerdezement bezeichnet) oder Portlandzement), Metalle, wie Aluminium, Ruß, ferner Holz, mineralische oder organische Fasern, oder dergleichen, oder Gemische von zwei oder mehr davon Verwendung. Die Füllstoffe können in beliebigen Formen vorliegen, beispielsweise als Pulver oder Mehl, oder als Formkörper, z.B. in Zylinder-, Ring-, Kugel-, Plättchen-, Stäbchen-, Sattel- oder Kristallform, oder ferner in Faserform (fibrilläre Füllstoffe), und die entsprechenden Grundteilchen haben vorzugsweise einen maximalen Durchmesser von etwa 10 mm und einen Minimaldurchmesser von etwa 1 nm. Das heißt, der Durchmesser ist etwa 10 mm oder irgendein Wert von weniger als etwa 10 mm, aber mehr als etwa 1 nm. Bevorzugt ist der maximale Durchmesser ein Durchmesser von etwa 5 mm, bevorzugter von etwa 3 mm, noch bevorzugter von etwa 0,7 mm. Ganz besonders bevorzugt ist ein maximaler Durchmesser von etwa 0,5 mm. Der bevorzugtere Minimaldurchmesser ist etwa 10 nm, noch bevorzugter etwa 50 nm, ganz besonders bevorzugt etwa 100 nm. Durchmesserbereiche, die sich durch Kombination dieser maximalen Durchmesser und Minimaldurchmesser ergeben, sind besonders bevorzugt. Bevorzugt und deutlicher verstärkend wirken sich jedoch die globulären, inerten Stoffe (Kugelform) aus. Auch Core-Shell-Partikel, bevorzugt in Kugelform, können als Füllstoffe verwendet werden.

Bevorzugte Füllstoffe sind ausgewählt aus der Gruppe bestehend aus Zement, Kieselsäure, Quarz, Quarzsand, Quarzmehl, und Mischungen aus zwei oder mehreren davon. Für die Reaktivharzkomponente (A) besonders bevorzugt sind Füllstoffe ausgewählt aus der Gruppe bestehend aus Zement, pyrogener Kieselsäure, insbesondere unbehandelter, polarer pyrogener Kieselsäure, Quarzsand, Quarzmehl, und Mischungen aus zwei oder mehreren davon. Ganz besonders bevorzugt ist für die Reaktivharzkomponente (A) eine Mischung aus Zement (insbesondere Aluminatzement (oft auch als Tonerdezement bezeichnet) oder Portlandzement), pyrogener Kieselsäure und Quarzsand. Für die Härterkomponente (B) ist pyrogene Kieselsäure als alleiniger Füllstoff oder als einer von mehreren Füllstoffen bevorzugt; besonders bevorzugt sind neben der pyrogenen Kieselsäure noch einer oder mehrere weitere Füllstoffe vorhanden.

Als Additive finden übliche Additive Verwendung, also Thixotropiermittel, wie gegebenenfalls organisch oder anorganisch nachbehandelte pyrogene Kieselsäure (falls sie nicht schon als Füllstoff verwendet wird), insbesondere apolar nachbehandelte pyrogene Kieselsäure, Bentonite, Alkyl- und Methylcellulosen, Rhizinusölderivate oder dergleichen, Weichmacher, wie Phthalsäure- oder Sebacinsäureester, weitere Stabilisatoren neben den erfindungsgemäß verwendeten Stabilisatoren und Inhibitoren, Antistatikmittel, Verdickungsmittel, Flexibilisatoren, Rheologiehilfsmittel, Netzmittel, färbende Zusätze, wie Farbstoffe oder insbesondere Pigmente, beispielsweise zum unterschiedlichen Anfärben der Komponenten zur besseren Kontrolle von deren Durchmischung, oder dergleichen, oder Gemische von zwei oder mehr davon. Auch nicht reaktive Verdünnungsmittel (Lösungsmittel) können, vorzugsweise in einer Menge bis zu 30 Gew.-%, bezogen auf die Gesamtmenge der Reaktivharzkomponente enthalten sein, wie Niederalkylketone, z.B. Aceton, Diniederalkyl-niederalkanoylamide, wie Dimethylacetamid, Niederalkylbenzole, wie Xylole oder Toluol, Phthalsäureester oder Paraffine, Wasser oder Glykole. Ferner können Metallfänger in Form von oberflächenmodifizierten pyrogenen Kieselsäuren in der Reaktivharzkomponente enthalten sein. Bevorzugt ist mindestens ein Thixotropiermittel als Additiv vorhanden, besonders bevorzugt eine organisch oder anorganisch nachbehandelte pyrogene Kieselsäure, ganz besonders bevorzugt eine apolar nachbehandelte pyrogene Kieselsäure.

In dieser Hinsicht wird Bezug genommen auf die Anmeldungen WO 02/079341 und WO 02/079293 sowie WO 2011/128061 A1.

Der Anteil der Additive in der Reaktivharzkomponente kann bis zu etwa 5 Gew.-%, bezogen auf die Reaktivharzkomponente, betragen.

Die erfindungsgemäß hergestellten Reaktivharze sind in vielen Bereichen, bei denen sonst üblicherweise ungesättigte Polyesterharze, Vinylesterharze oder Vinylesterurethanharze Verwendung finden, einsetzbar. Sie finden üblicherweise Verwendung als Harzbestandteil in der Reaktivharzkomponente eines Reaktivharz-Systems, wie ein Mehrkomponenten-System, typischerweise ein Zweikomponenten-System aus einer Reaktivharzkomponente (A) und einer Härterkomponente (B). Dieses Mehrkomponenten-System kann in Form eines Patronen-Systems, eines Kartuschen-Systems oder eines Folienbeutel-Systems vorliegen. Bei der bestimmungsgemäßen Verwendung des Systems werden die Komponenten entweder unter Einwirkung mechanischer Kräfte oder durch Gasdruck aus den Patronen, Kartuschen oder Folienbeuteln ausgepresst, miteinander vermischt, vorzugsweise mit Hilfe eines Statikmischers, durch den die Bestandteile hindurchgeführt werden, und appliziert.

Ein Reaktivharz-System kann mit einer eben beschriebenen Reaktivharzkomponente (A) und einer Härterkomponente (B) formuliert werden, wobei (B) einen Initiator für die Epoxymethacrylat-Verbindung enthält.

Der Initiator ist gewöhnlich ein Peroxid. Alle dem Fachmann bekannten Peroxide, die zum Härten von ungesättigten Polyesterharzen und Vinylesterharzen verwendet werden, können eingesetzt werden. Derartige Peroxide umfassen organische und anorganische Peroxide, entweder flüssig oder fest, wobei Wasserstoffperoxid auch verwendet werden kann. Beispiele geeigneter Peroxide sind Peroxycarbonate (der Formel -OC(O)O-), Peroxyester (der Formel -C(O)OO-), Diacylperoxide (der Formel -C(O)OOC(O)-), Dialkylperoxide (der Formel -OO-) und dergleichen. Diese können als Oligomer oder Polymer vorliegen.

Bevorzugt sind die Peroxide aus der Gruppe der organischen Peroxide ausgewählt. Geeignete organische Peroxide sind: tertiäre Alkylhydroperoxide, wie tert-Butylhydroperoxid, und andere Hydroperoxide, wie Cumenhydroperoxid, Peroxyester oder Persäuren, wie tert-Butylperester, Benzoylperoxid, Peracetate und Perbenzoate, Laurylperoxid, einschließlich (Di)peroxyesters, Perether, wie Peroxydiethylether, Perketone, wie Methylethylketonperoxid. Die als Härter verwendeten organischen Peroxide sind oft tertiäre Perester oder tertiäre Hydroperoxide, d.h. PeroxidVerbindungen mit tertiären Kohlenstoffatomen, die direkt an eine -O-O-acyl- oder -OOH-Gruppe gebunden ist. Aber auch Gemische dieser Peroxide mit anderen Peroxiden können erfindungsgemäß eingesetzt werden. Die Peroxide können auch gemischte Peroxide sein, d.h. Peroxide, die zwei verschiedene Peroxid-tragende Einheiten in einem Molekül aufweisen. Bevorzugt wird zum Härten (Di-benzoyl)peroxid (BPO) verwendet.

Das Reaktivharz-System kann in Form eines Zwei- oder Mehrkomponenten-Systems vorliegen, bei dem die jeweiligen Komponenten räumlich getrennt voneinander vorliegen, so dass eine Reaktion (Aushärtung) der Komponenten erst nach deren Vermischen erfolgt.

Ein Zweikomponenten-Reaktivharz-System umfasst bevorzugt die A Komponente und die B Komponente reaktionsinhibierend getrennt in unterschiedlichen Behältern, beispielsweise einer Mehrkammer-Vorrichtung, wie eine Mehrkammer-Patrone und/oder -Kartusche, aus welchen Behältern die beiden Komponenten durch Einwirkung mechanischer Presskräfte oder unter Einwirkung eines Gasdruckes ausgepresst und vermischt werden. Eine weitere Möglichkeit besteht darin, das Zweikomponenten-Reaktivharzsystem als Zweikomponenten-Kapseln zu konfektionieren, die in das Bohrloch eingeführt werden und durch schlagdrehendes Setzen des Befestigungselements zerstört werden unter gleichzeitiger Durchmischung der beiden Komponenten der Mörtelmasse. Vorzugsweise wird hierin ein Patronensystem oder ein Injektionssystem eingesetzt, bei dem die beiden Komponenten aus den getrennten Behältern ausgepresst und durch einen statischen Mischer geführt werden, in dem sie homogen vermischt und dann über eine Düse vorzugsweise direkt in das Bohrloch ausgetragen werden.

Bei einer bevorzugten Ausführungsform ist das Reaktivharz-System ein Zweikomponenten-System und enthält die Reaktivharzkomponente (A) neben dem Backbone-Harz zusätzlich noch eine hydraulisch abbindende oder polykondensierbare anorganische Verbindung, insbesondere Zement, und die Härterkomponente (B) neben dem Initiator für die Polymerisation des Backbone-Harzes noch Wasser. Derartige Hybridmörtelsysteme sind ausführlich in DE 4231161 A1 beschrieben. Dabei enthält die Komponente (A) vorzugsweise als hydraulisch abbindende oder polykondensierbare anorganische Verbindung Zement, beispielsweise Portlandzement oder Tonerdezement, wobei übergangsmetalloxidfreie oder übergangsmetallarme Zemente besonders bevorzugt sind. Als hydraulisch abbindende anorganische Verbindung kann auch Gips als solcher oder in Mischung mit dem Zement eingesetzt werden. Die Komponente (A) kann als polykondensierbare anorganische Verbindung auch silikatische, polykondensierbare Verbindungen, insbesondere lösliches, gelöstes und/oder amorphes Siliziumdioxid enthaltende Stoffe wie z.B. polare, nicht nachbehandelte pyrogene Kieselsäure umfassen.

Das Volumenverhältnis von Komponente A zu Komponente B in einem Zweikomponenten-System ist vorzugsweise 3:1; 5:1 oder 7:1. Besonders bevorzugt ist ein Volumenverhältnis 3:1 oder 5:1.

In einer bevorzugten Ausführungsform enthält die Reaktivharzkomponente (A) also folglich:
- mindestens ein wie oben definiertes Epoxymethacrylat, bevorzugt eine Verbindung der Formel (II), (III), (IV), (V) oder (VI);
- mindestens einen wie oben definierten Inhibitor vom Typ Piperidinyl-N-oxyl oder Tetrahydropyrrol-N-oxyl, bevorzugt TEMPOL;
- mindestens einen wie oben definierten Beschleuniger, bevorzugt ein Toluidin-Derivat, besonders bevorzugt Di-iso-propanol-p-toluidin;
- mindestens eine hydraulisch abbindende oder polykondensierbare anorganische Verbindung, bevorzugt Zement; und
- mindestens ein Thixotropiermittel, bevorzugt pyrogene Kieselsäure,
und die Härterkomponente (B) enthält:
- mindestens einen Initiator für die Initiierung der Polymerisation des Epoxymethacrylats, bevorzugt Benzoylperoxid (BPO) oder tert-Butylperoxybenzoat; und
- Wasser.

In einer bevorzugteren Ausführungsform enthält die Reaktivharzkomponente (A):
- mindestens ein wie oben definiertes Epoxymethacrylat, bevorzugt eine Verbindung der Formel (II), (III), (IV), (V) oder (VI);
- mindestens einen wie oben definierten Inhibitor vom Typ Piperidinyl-N-oxyl oder Tetrahydropyrrol-N-oxyl, bevorzugt TEMPOL;
- mindestens einen Beschleuniger, bevorzugt ein Toluidin-Derivat, besonders bevorzugt Di-iso-propanol-p-toluidin;
- mindestens eine hydraulisch abbindende oder polykondensierbare anorganische Verbindung, bevorzugt Zement; und
- mindestens ein Thixotropiermittel, bevorzugt pyrogene Kieselsäure,
und die Härterkomponente (B) enthält:
- mindestens einen Initiator für die Initiierung der Polymerisation des Epoxymethacrylats, bevorzugt Benzoylperoxid (BPO) oder tert-Butylperoxybenzoat;
- mindestens einen Füllstoff, bevorzugt Quarzsand oder Quarzmehl; und
- Wasser.

In einer noch bevorzugteren Ausführungsform enthält die Reaktivharzkomponente (A):
- mindestens ein wie oben definiertes Epoxymethacrylat, bevorzugt eine Verbindung der Formel (II), (III), (IV), (V) oder (VI);
- mindestens einen wie oben definierten Inhibitor vom Typ Piperidinyl-*N*-oxyl oder Tetrahydropyrrol-*N*-oxyl, bevorzugt TEMPOL;
- mindestens einen Beschleuniger, bevorzugt ein Toluidin-Derivat, besonders bevorzugt Di-*iso*-propanol-*p*-toluidin;
- mindestens einen weiteren Inhibitor, der ausgewählt ist aus der Gruppe besteht aus Catecholen und Phenothiazinen;
- mindestens eine hydraulisch abbindende oder polykondensierbare anorganische Verbindung, bevorzugt Zement; und
- mindestens ein Thixotropiermittel, bevorzugt pyrogene Kieselsäure,
und die Härterkomponente (B) enthält:
- mindestens einen Initiator für die Initiierung der Polymerisation des Epoxymethacrylats, bevorzugt Benzoylperoxid (BPO) oder tert-Butylperoxybenzoat;
- mindestens einen Füllstoff, bevorzugt Quarzsand oder Quarzmehl;
- mindestens ein Thixotropiermittel, bevorzugt pyrogene Kieselsäure; und
- Wasser.

In einer noch bevorzugteren Ausführungsform enthält die Reaktivharzkomponente (A):
- mindestens ein wie oben definiertes Epoxymethacrylat, bevorzugt eine Verbindung der Formel (II), (III), (IV), (V) oder (VI);
- mindestens einen wie oben definierten Inhibitor vom Typ Piperidinyl-*N*-oxyl oder Tetrahydropyrrol-*N*-oxyl, bevorzugt TEMPOL;
- mindestens einen Beschleuniger, bevorzugt ein Toluidin-Derivat, besonders bevorzugt Di-*iso*-propanol-*p*-toluidin;
- mindestens einen weiteren Inhibitor, der ausgewählt ist aus der Gruppe besteht aus Catecholen und Phenothiazinen;
- mindestens eine hydraulisch abbindende oder polykondensierbare anorganische Verbindung, bevorzugt Zement;
- mindestens ein Thixotropiermittel, bevorzugt pyrogene Kieselsäure, und
- mindestens einen weiteren Füllstoff, bevorzugt Quarzsand,
und die Härterkomponente (B) enthält:
- Benzoylperoxid (BPO) odertert-Butylperoxybenzoat als Initiator für die Initiierung der Polymerisation des Epoxymethacrylats;
- mindestens einen Füllstoff, bevorzugt Quarzsand oder Quarzmehl;
- mindestens ein Thixotropiermittel, bevorzugt pyrogene Kieselsäure; und
- Wasser.

In einer noch bevorzugteren Ausführungsform enthält die Reaktivharzkomponente (A):
- mindestens ein wie oben definiertes Epoxymethacrylat, bevorzugt eine Verbindung der Formel (II), (III), (IV), (V) oder (VI);
- TEMPOL;
- Di-*iso-*propanol-*p*-toluidin;
- mindestens einen weiteren Inhibitor, der ausgewählt ist aus der Gruppe besteht aus Catecholen und Phenothiazinen;
- Zement;
- pyrogene Kieselsäure; und
- Quarzsand,
und die Härterkomponente (B) enthält:
- mindestens einen Initiator für die Initiierung der Polymerisation des Epoxymethacrylats;
- pyrogene Kieselsäure;
- Quarzsand oder Quarzmehl und
- Wasser.

In jeder dieser Ausführungsformen enthält in einer bevorzugten Ausführungsform die Reaktivharzkomponente (A) zusätzlich noch mindestens einen Reaktivverdünner. Bevorzugt ist dieser Reaktivverdünner ein Monomer oder eine Mischung aus mehreren Monomeren des Backbone-Harzes.

Die Reaktivharzkomponenten (A) und die Härterkomponenten (B) in jeder dieser Ausführungsformen sind beliebig miteinander kombinierbar.

Ein solches Reaktivharz-System findet vor allem im Baubereich Verwendung (bauliche Zwecke), beispielsweise bei der Erstellung und Instandhaltung bzw. -setzung von Bauteilen und Bauwerken, etwa aus Beton, als Polymerbeton, als Beschichtungsmasse auf Kunstharzbasis oder als kalthärtende Straßenmarkierung, zur Verstärkung von Bauteilen und Bauwerken, beispielsweise Wände, Decken oder Böden, die Befestigung von Bauteilen, wie Platten oder Blöcken, z. B. aus Stein, Glas oder Kunststoff, an Bauteilen oder Bauwerken, etwa durch Kleben (bauliches Kleben). Besonders eignet sie sich zur chemischen Befestigung. Ganz besonders eignet sie sich zur (stoff- und/oder formschlüssigen) chemischen Befestigung von Verankerungsmitteln, wie Ankerstangen, Bolzen, Bewehrungseisen, Schrauben oder dergleichen in Aussparungen, wie Bohrlöchern, insbesondere in Bohrlöchern in verschiedenen Untergründen, insbesondere mineralischen Untergründen, wie solche auf der Grundlage von Beton, Porenbeton, Ziegelwerk, Kalksandstein, Sandstein, Naturstein, Glas und dergleichen, und metallischen Untergründen, wie solche aus Stahl. In einer Ausführungsform ist der Untergrund des Bohrlochs Beton, und das Verankerungsmittel besteht aus Stahl oder Eisen. In einer weiteren Ausführungsform ist der Untergrund des Bohrlochs Stahl, und das Verankerungsmittel besteht aus Stahl oder Eisen. Hierzu werden die Komponenten in das Bohrloch injiziert, wonach die zu befestigenden Einrichtungen, wie Ankergewindestangen und dergleichen, in das mit dem aushärtenden Reaktivharz beschickte Bohrloch eingebracht und entsprechend justiert werden.

Die folgenden Beispiele dienen der weiteren Erläuterung der Erfindung.

### BEISPIELE

Es wurden Reaktivharz-Masterbatches, Reaktivharze, Reaktivharzkomponenten und Zweikomponenten-Reaktivharz-Systeme unter Verwendung der Verbindungen (II) und (VI) als Backbone-Harz hergestellt. Die dynamische Viskosität der Reaktivharze und der Reaktivharzkomponenten und die Auspresskräfte der Zweikomponenten-Reaktivharz-Systeme wurden ermittelt.

### A1. Herstellung des Reaktivharz-Masterbatches A1 mit Verbindung (II)

In einem 2 Liter Laborglasreaktor mit Innenthermometer und Rührwelle wurden 645 g 1,4-Butandiol-diglycidylether (Araldite^{®} DY 026 SP; Huntsmann Advanced Materials), 518 g Methacrylsäure (BASF SE), 6,0 g Tetraethylammoniumbromid (Merck KGaA Deutschland), 0,23 g Phenothiazin (D Prills; Allessa Chemie) und 0,25 g 4-Hydroxy-2,2,6,6-tetramethyl-piperidinyl-1-oxyl (TEMPOL; Evonik Industries AG) gegeben. Der Ansatz wurde für 240 Minuten auf 100°C erwärmt.

Hierdurch wurde der Reaktivharz-Masterbatch A1 erhalten, der die Verbindung (II) als Backbone-Harz und enthält. Die Verbindung (II) weist folgende Struktur auf:

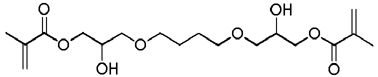

### A2. Herstellung des Reaktivharzes A2

6,5 g 4-Hydroxy-2,2,6,6-tetramethyl-piperidinyl-1-oxyl (TEMPOL; Evonik Degussa GmbH) und 26,25 g Di-iso-propanol-p-toluidin (BASF SE) wurden zu einer Mischung aus 489 g Reaktivharz-Masterbatch A1, 489 g Hydroxypropylmethacrylat und 489 g 1,4-Butandioldimethacrylat (BDDMA; Evonik AG) gegeben.

Hierdurch wurde das Reaktivharz A2 erhalten, das die Verbindung (II) als Backbone-Harz enthält.

### A3. Herstellung der Reaktivharzkomponente A3

354 g Reaktivharz A2 wurden mit 185 g Secar^{®} 80 (Kerneos Inc.), 27 g CAB-O-SIL^{®} TS-720 (Cabot Corporation) und 335 g Quarzsand F32 (Quarzwerke GmbH) im Dissolver unter Vakuum mit einem PC Laborsystem Dissolver vom Typ LDV 0.3-1 vermischt. Die Masse wurde für 8 Minuten bei 3500 U/min unter Vakuum (p≤100 mbar) mit einer 55 mm Dissolverscheibe und einem Randabstreifer dezentral gerührt.

Hierdurch wurde die Reaktivharzkomponente A3 erhalten.

### B1. Herstellung des Reaktivharz-Masterbatches B1 mit Verbindung (VI)

In einem 2 Liter Laborglasreaktor mit Innenthermometer und Rührwelle wurden 840 g

| | | | |
|---|---|---|---|
| Trimethylolpropantriglycidylether, | 742 g | Methacrylsäure, | 17,5 g |

Tetraethylammoniumbromid, 0,33 g Phenothiazin (D Prills; Allessa Chemie) und 0,36 g 4-Hydroxy-2,2,6,6-tetramethyl-piperidinyl-1-oxyl (TEMPOL; Evonik Degussa GmbH) gegeben. Der Ansatz wurde für 300 Minuten auf 100°C erwärmt. Anschließend wurden 400 g 1,4-Butandioldimethacrylat (BDDMA; Evonik AG) zugegeben.

Hierdurch wurde der Reaktivharz-Masterbatch B1 erhalten, der die Verbindung (VI) als Backbone-Harz enthält. Die Verbindung (VI) hat folgende Struktur:

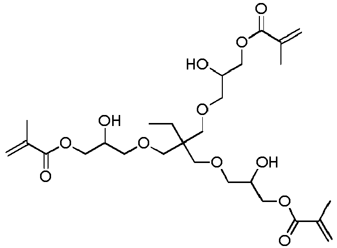

### B2. Herstellung des Reaktivharzes B2

6,0 g 4-Hydroxy-2,2,6,6-tetramethyl-piperidinyl-1-oxyl (TEMPOL; Evonik Degussa GmbH) und 22,8 g Diisopropanol-p-toluidin (BASF SE) wurden zu einer Mischung aus 530 g Reaktivharz-Masterbatch B1, 424 g Hydroxypropylmethacrylat und 318 g 1,4-Butandioldimethacrylat (BDDMA; Evonik AG) gegeben.

Hierdurch wurde das Reaktivharz B2 erhalten, das die Verbindung (VI) als Backbone-Harz enthält.

### B3. Herstellung der Reaktivharzkomponente B3

354 g Reaktivharz B2 wurden mit 185 g Secar^{®} 80 (Kerneos Inc.), 27 g CAB-O-SIL^{®} TS-720 (Cabot Corporation) und 335 g Quarzsand F32 (Quarzwerke GmbH) im Dissolver unter Vakuum mit einem PC Laborsystem Dissolver vom Typ LDV 0.3-1, wie unter Punkt A3 angegeben, vermischt.

Hierdurch wurde die Reaktivharzkomponente B3 erhalten.

### C1. Herstellung des Vergleichsreaktivharz-Masterbatches mit Vergleichsverbindung 1

Der Vergleichsreaktivharz-Masterbatch C1, der die Vergleichsverbindung 1 als Backbone-Harz enthält, wurde gemäß dem Verfahren in EP 0 713 015 A1, die hiermit als Referenz eingeführt und auf deren gesamte Offenbarung verwiesen wird, synthetisiert.

Hierdurch wurde der Vergleichsreaktivharz-Masterbatch C1 erhalten, der 65 Gew.-% Vergleichsverbindung 1 als Backbone-Harz und 35 Gew.-% Hydroxypropylmethacrylat, bezogen auf das Gesamtgewicht des Vergleichsreaktivharz-Masterbatches, enthält.

Das Produkt (Vergleichsverbindung 1) hat eine Oligomerverteilung, wobei das Oligomer mit einer Wiederholeinheit folgende Struktur hat:

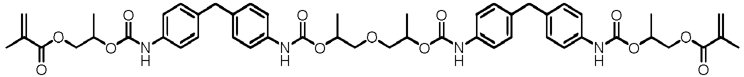

### C2. Herstellung des Vergleichsreaktivharzes C2

9,2 g 4-Hydroxy-2,2,6,6-tetramethyl-piperidinyl-1-oxyl (TEMPOL; Evonik Industries AG) und 35,0 g Di-iso-propanol-p-toluidin (BASF SE) wurden zu einer Mischung aus 1004 g Vergleichsreaktivharz-Masterbatch C1, 300 g Hydroxypropylmethacrylat und 652 g 1,4-Butandioldimethacrylat (BDDMA; Evonik AG) gegeben.

Hierdurch wurde das Vergleichsreaktivharz C2 erhalten, das die Vergleichsverbindung 1 als Backbone-Harz enthält.

### C3. Herstellung der Vergleichsreaktivharzkomponente C3

354 g Vergleichsreaktivharz C2 wurden mit 185 g Secar^{®} 80 (Kerneos Inc.), 27 g CAB-O-SIL^{®} TS-720 (Cabot Corporation) und 335 g Quarzsand F32 (Quarzwerke GmbH) im Dissolver unter Vakuum mit einem PC Laborsystem Dissolver vom Typ LDV 0.3-1, wie unter Punkt A3 angegeben, vermischt.

Hierdurch wurde die Vergleichsreaktivharzkomponente C3 erhalten.

Um den Einfluss der Verbindungen (II) und (VI) auf die Viskosität eines diese Verbindungen enthaltenden Reaktivharz-Masterbatches, eines Reaktivharzes und einer Reaktivharzkomponente zu zeigen, wurden die Viskosität der erfindungsgemäßen Reaktivharzkomponenten sowie die Auspresskräfte von Zweikomponenten-Reaktivharz-Systemen gemessen und jeweils mit der Vergleichsreaktivharzkomponente und dem Vergleichszweikomponenten-Reaktivharz-System verglichen.

### Messung der dynamischen Viskosität der Reaktivharze

Die Messung der dynamischen Viskosität der Reaktivharze A2 und B2 und des Vergleichsreaktivharzes C2 erfolgte mit einem Kegel-Platte Messsystem nach DIN 53019. Der Durchmesser des Kegels betrug 60mm und der Öffnungswinkel ist 1°. Es wurde bei einer konstanten Schergeschwindigkeit von 150/s und einer Temperatur von 23°C gemessen (wenn keine abweichende Angabe bei den Messdaten gemacht wird). Die Messdauer betrug 180s und jede Sekunde wurde ein Messpunkt generiert. Zur Erreichung der Schergeschwindigkeit wurde eine Rampe von 0-150/s mit einer Dauer von 120s vorgeschaltet. Da es sich um newtonschen Flüssigkeiten handelt, wurde über den Messabschnitt mit konstanter Schergeschwindigkeit von 150/s eine lineare Auswertung über den Messabschnitt vorgenommen und die Viskosität bestimmt. Es wurden jeweils drei Messungen durchgeführt, wobei die in Tabelle 1 angegebenen Werte der Mittelwert der drei Messungen ist.

### Messung der dynamischen Viskosität der Reaktivharzkomponenten

Die Messung der dynamischen Viskosität der Reaktivharzkomponenten A3 und B3 und der Vergleichsreaktivharzkomponente C3 erfolgte mit einem Platte-Platte Messsystem nach DIN 53019. Der Durchmesser der Platte betrug 20mm und der Spaltabstand betrug 3mm. Um ein Austreten der Probe aus dem Spalt zu verhindern, wurde ein Begrenzungsring aus Teflon verwendet, der einen Abstand von 1mm von der oberen Platte hatte. Die Messtemperatur betrug 25°C. Die Methode bestand aus drei Abschnitten: 1. Niedrige Scherung, 2. Hohe Scherung, 3. Niedrige Scherung. Beim 1. Abschnitt wurde 3 Minuten bei 0,5/s geschert. Im 2. Abschnitt wurde in 8 Stufen zu je 15 Sekunden logarithmisch die Schergeschwindigkeit von 0,8/s auf 100/s gesteigert. Die einzelnen Stufen dabei waren: 0,8/s; 1,724/s; 3,713/s; 8/s; 17,24/s; 37,13/s; 80/s; 100/s. Der 3. Abschnitt war die Wiederholung des 1 Abschnitts. Am Ende eines jeden Abschnittes wurden die Viskositäten ausgelesen. Die in Tabelle 2 zusammengefassten Werte entsprechen dem Wert des zweiten Abschnittes bei 100/s. Es wurden jeweils drei Messungen durchgeführt, wobei die in Tabelle 2 angegebenen Werte der Mittelwert der drei Messungen ist.

### Messung der der Auspresskräfte der Zweikomponenten-Reaktivharz-Systeme

Zur Bestimmung der Auspresskräfte bei 0°C und 25°C wurden die wie oben hergestellten Reaktivharzkomponenten (Komponente (A)) und die Härterkomponente (Komponente (B)) des kommerziell erhältlichen Produkts HIT-HY 110 (Hilti Aktiengesellschaft; Chargennummer: 1610264) in Plastikkartuschen (Firma Ritter GmbH; Volumenverhältnis A:B = 3:1) mit den Innendurchmessern circa 47 mm (Komponente (A)) bzw. circa 28 mm (Komponente (B)) abgefüllt und auf 0°C bzw. 25°C temperiert. Die Kartuschen wurden auf einer Material-Prüfmaschine der Firma Zwick mit einem Kraftaufnehmer (Prüfbereich bis 10 kN) über einen Statikmischer (Mischer HIT-RE-M; Hilti Aktiengesellschaft) von mit einer konstanten Geschwindigkeit von 100 mm/min über eine Strecke von 45 mm ausgepresst und dabei die mittlere auftretende Kraft gemessen.

Die dynamische Viskosität der Reaktivharze A2 und B2 wurde mit der dynamischen Viskosität des Vergleichsreaktivharzes C2 verglichen. Die Ergebnisse sind in Tabelle 1 zusammengefasst.

**Tabelle 1: Ergebnisse der Messung der dynamischen Viskosität der Reaktivharze A2 und B2 und des Vergleichsreaktivharzes C2**

| | **Reaktivharz A2** | **Reaktivharz B2** | **Vergleichsreaktivharz C2** |
|---|---|---|---|
| **Viskosität [mPa·s]** | 22 | 28 | 70 |

Aus den Werten in Tabelle 1 ist ersichtlich, dass die Reaktivharze A2 und B2, welche erfindungsgemäß die Verbindungen (II) und (VI) als Backbone-Harz enthalten, eine deutliche niedrigere dynamische Viskosität haben verglichen mit der dynamischen Viskosität des Vergleichsharzes C2, das die Vergleichsverbindung 1 als Backbone-Harz enthält.

Die dynamische Viskosität der Reaktivharzkomponenten A3 und B3 wurde mit der dynamischen Viskosität der Vergleichsreaktivharzkomponente C3 verglichen. Die Messwerte sind in Tabelle 2 zusammengefasst.

**Tabelle 2: Ergebnisse der Messung der dynamischen Viskosität der Reaktivharzkomponenten A3 und B3 und der Vergleichsreaktivharzkomponente C3**

| | **Reaktivharzkomponente A3** | **Reaktivharzkomponente B3** | **Vergleichsreaktivharzkomponente C3** |
|---|---|---|---|
| **Viskosität [Pa·s]** | 11,3 | 12,2 | 13,9 |

Die Werte in Tabelle 2 zeigen, dass auch die aus den Reaktivharzen A2 und B2 hergestellten Reaktivharzkomponenten A3 und B3 eine niedrige dynamische Viskosität haben, verglichen mit der dynamischen Viskosität der Vergleichskomponente C3 aus dem Vergleichsreaktivharz C2.

Die Auspresskräfte der Zweikomponenten-Reaktivharz-Systeme, die die erfindungsgemäßen Reaktivharzkomponenten A3 und B4 enthalten, wurden mit der Auspresskraft des Vergleichszweikomponenten-Reaktivharz-Systems, das die Vergleichsreaktivharzkomponente C3 enthält, verglichen. Die Messwerte bei 0°C und bei 25°C sind in Tabelle 3 zusammengefasst.

**Tabelle 3: Auspresskräfte der Zweikomponenten-Reaktivharz-Systeme mit den Reaktivharzkomponenten A3 und B3 und des Vergleichszweikomponenten-Reaktivharz-Systems mit der Vergleichsreaktivharzkomponente C3 bei 0°C und bei 25°C**

| | **Reaktivharz-System mit Reaktivharzkomponente A3** | **Reaktivharz-System mit Reaktivharzkomponente B3** | **Vergleichsreaktivharz-System mit Vergleichsreaktivharzkomponente C3** |
|---|---|---|---|
| **Kraft bei 0°C [N]** | 1203 | 1270 | 1631 |
| **Kraft bei 25°C [N]** | 843 | 959 | 1151 |

Die Ergebnisse in Tabelle 3 zeigen, dass die Zweikomponenten-Reaktivharz-Systeme, welche erfindungsgemäß die Verbindungen (II) und (VI) als Backbone-Harze enthalten, sowohl bei 25°C als auch bei 0°C deutlich niedrigere Auspresskräfte aufweisen als das Vergleichszweikomponenten-Reaktivharz-System, welches die Vergleichsverbindung 1 als Backbone-Harz enthält.

## Patentansprüche

1. Verwendung einer Verbindung der allgemeinen Formel (I) 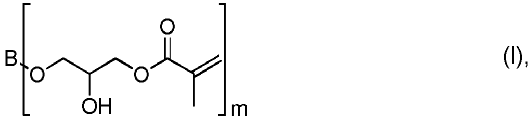 worin
m eine ganze Zahl größer oder gleich 2 ist, und
B eine lineare, verzweigte oder cyclische aliphatische Kohlenwasserstoffgruppe ist, die gegebenenfalls substituiert sein kann,
in einem Reaktivharz oder einer Reaktivharzkomponente für die chemische Befestigung von Verankerungsmitteln in Aussparungen

2. Verwendung nach Anspruch 1, wobei die aliphatische Kohlenwasserstoffgruppe B substituiert ist.

3. Verwendung nach Anspruch 2, wobei die aliphatische Kohlenwasserstoffgruppe B hydroxysubstituiert ist.

4. Verwendung nach einem der vorhergehenden Ansprüche, wobei die aliphatische Kohlenwasserstoffgruppe eine lineare oder verzweigte C₂-C₁₂-Alkylengruppe ist.

5. Verwendung nach Anspruch 4, wobei die aliphatische Kohlenwasserstoffgruppe eine lineare oder verzweigte C₂-C₈-Alkylengruppe ist.

6. Verwendung nach einem der vorhergehenden Ansprüche, wobei m 2 oder 3 ist und die aliphatische Kohlenwasserstoffgruppe eine lineare oder verzweigte C₂-C₆-Alkylengruppe ist.

7. Verwendung einer Verbindung, wie in einem der Ansprüche 1 bis 6 definiert, zur Verringerung der Viskosität eines Reaktivharzes für bauliche Zwecke.

8. Verwendung einer Verbindung, wie in einem der Ansprüche 1 bis 6 definiert, zur Erniedrigung der Auspresskräfte einer diese Verbindung enthaltenden Reaktivharzkomponente oder eines diese Verbindung enthaltenden Reaktivharz-Systems.

9. Reaktivharz umfassend eine Verbindung, wie in einem der Ansprüche 1 bis 6 definiert, einen Inhibitor, einen Beschleuniger und gegebenenfalls einen Reaktivverdünner.

10. Verwendung eines Reaktivharzes nach Anspruch 9 für bauliche Zwecke.

11. Verwendung nach Anspruch 10 zur chemischen Befestigung von Verankerungsmitteln in Bohrlöchern.

## Claims

1. A use of a compound of general formula (I) 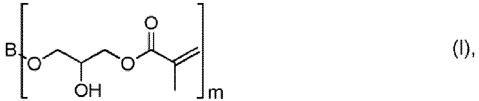 where
m is an integer greater than or equal to 2, and
B is a linear, branched or cyclic aliphatic hydrocarbon group which can optionally be substituted,
in a reactive resin or a reactive resin component for the chemical fastening of anchoring means in recesses.

2. The use according to claim 1, wherein the aliphatic hydrocarbon group B is substituted.

3. The use according to claim 2, wherein the aliphatic hydrocarbon group B is hydroxy-substituted.

4. The use according to any of the preceding claims, wherein the aliphatic hydrocarbon group is a linear or branched C₂-C₁₂ alkylene group.

5. The use according to claim 4, wherein the aliphatic hydrocarbon group is a linear or branched C₂-C₈ alkylene group.

6. The use according to any of the preceding claims, wherein m is 2 or 3 and the aliphatic hydrocarbon group is a linear or branched C₂-C₆ alkylene group.

7. A use of a compound as defined in any of claims 1 to 6 for reducing the viscosity of a reactive resin for construction purposes.

8. A use of a compound as defined in any of claims 1 to 6 for reducing the discharge forces of a reactive resin component containing this compound or of a reactive resin system containing this compound.

9. A reactive resin comprising a compound as defined in any of claims 1 to 6, an inhibitor, an accelerator and optionally a reactive diluent.

10. A use of a reactive resin according to claim 9 for construction purposes.

11. The use according to claim 10 for the chemical fastening of anchoring means in boreholes.

## Revendications

1. Utilisation d'un composé de formule générale (I) 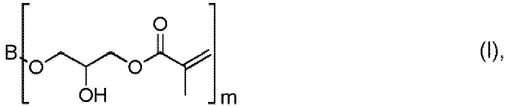 où
m représente un nombre entier supérieur ou égal à 2, et
B représente un groupe hydrocarboné aliphatique linéaire, ramifié ou cyclique, qui peut éventuellement être substitué,
dans une résine réactive ou un composant de résine réactive pour la fixation chimique de moyens d'ancrage dans des évidements.

2. Utilisation selon la revendication 1, dans laquelle le groupe hydrocarboné aliphatique B est substitué.

3. Utilisation selon la revendication 2, dans laquelle le groupe hydrocarboné aliphatique B est substitué par un groupe hydroxy.

4. Utilisation selon l'une quelconque des revendications précédentes, dans laquelle le groupe hydrocarboné aliphatique est un groupe alkylène en C₂-C₁₂ linéaire ou ramifié.

5. Utilisation selon la revendication 4, dans laquelle le groupe hydrocarboné aliphatique est un groupe alkylène en C₂-C₈ linéaire ou ramifié.

6. Utilisation selon l'une quelconque des revendications précédentes, dans laquelle m représente 2 ou 3 et le groupe hydrocarboné aliphatique représente un groupe alkylène en C₂-C₆ linéaire ou ramifié.

7. Utilisation d'un composé tel que défini dans l'une des revendications 1 à 6 pour la réduction de la viscosité d'une résine réactive à des fins de construction.

8. Utilisation d'un composé tel que défini dans l'une des revendications 1 à 6 pour la diminution des forces de pression d'un composant de résine réactive contenant ledit composé ou d'un système de résine réactive contenant ledit composé.

9. Résine réactive comprenant un composé tel que défini dans l'une des revendications 1 à 6, un inhibiteur, un accélérateur et éventuellement un diluant réactif.

10. Utilisation d'une résine réactive selon la revendication 9 à des fins de construction.

11. Utilisation selon la revendication 10 pour la fixation chimique de moyens d'ancrage dans des puits.
